(19) Europäisches Patentamt
European Patent Office
Office européen des brevets

(11) EP 1 235 809 B1

(12) **EUROPÄISCHE PATENTSCHRIFT**

(45) Veröffentlichungstag und Bekanntmachung des Hinweises auf die Patenterteilung:
**12.11.2003 Patentblatt 2003/46**

(21) Anmeldenummer: **00993781.4**

(22) Anmeldetag: **21.11.2000**

(51) Int Cl.[7]: **C07D 221/26**, C07D 221/22, C07D 407/06, A61K 31/445 // (C07D407/06, 307:00), C07D211:00

(86) Internationale Anmeldenummer:
**PCT/EP00/11585**

(87) Internationale Veröffentlichungsnummer:
**WO 01/051471 (19.07.2001 Gazette 2001/29)**

(54) **SUBSTITUIERTE 1,2,3,4,5,6-HEXAHYDRO-2,6-METHANO-3-BENZAZOCINE UND IHRE VERWENDUNG ALS ARZNEIMITTEL**

SUBSTITUTED 1,2,3,4,5,6-HEXAHYDRO-2,6-METHANO-3-BENZAZOCINES AND THE USE THEREOF AS MEDICAMENTS

1,2,3,4,5,6-HEXAHYDRO-2,6-METHANO-3-BENZAZOCINE SUBSTITUEE ET SON UTILISATION COMME PRODUIT PHARMACEUTIQUE

(84) Benannte Vertragsstaaten:
**AT BE CH CY DE DK ES FI FR GB GR IE IT LI LU MC NL PT SE TR**
Benannte Erstreckungsstaaten:
**LT LV RO SI**

(30) Priorität: **27.11.1999 DE 19957156**

(43) Veröffentlichungstag der Anmeldung:
**04.09.2002 Patentblatt 2002/36**

(73) Patentinhaber: **Boehringer Ingelheim Pharma GmbH & Co.KG**
**55218 Ingelheim am Rhein (DE)**

(72) Erfinder:
- **GRAUERT, Matthias**
  **55218 Ingelheim am Rhein (DE)**
- **CARTER, Adrian**
  **55411 Bingen am Rhein (DE)**
- **WEISER, Thomas**
  **55268 Nieder-Olm (DE)**
- **ENSINGER, Helmut**
  **55218 Ingelheim am Rhein (DE)**
- **GAIDA, Wolfram**
  **55218 Ingelheim am Rhein (DE)**
- **MIERAU, Joachim**
  **55127 Mainz (DE)**

(56) Entgegenhaltungen:
**EP-A- 0 521 422**
**WO-A-00/50421**
**WO-A-99/14199**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 235 809 B1

Printed by Jouve, 75001 PARIS (FR)

## Beschreibung

Gegenstand der vorliegenden Erfindung sind substituierte 1,2,3,4,5,6-Hexahydro-2,6-methano-3-benzazocine der allgemeinen Formel **1**

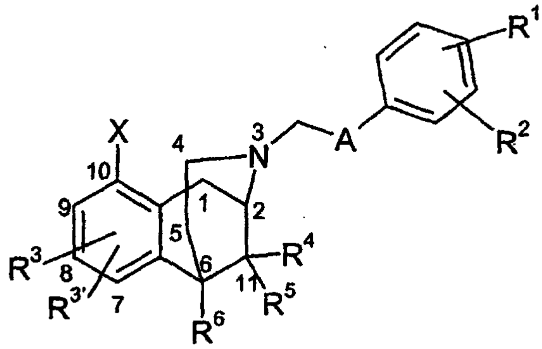

**1**

worin

| | |
|---|---|
| $R^1$und $R^2$ | gleich oder verschieden Wasserstoff, $C_1$-$C_6$-Alkyl, $C_1$-$C_6$-Alkyloxy, OH, F, Cl oder Br; |
| $R^3$ und $R^{3'}$ | gleich oder verschieden Wasserstoff, F, Cl, Br, Methyl, Ethyl, OH, $CF_3$, Methoxy oder Phenyl, welches gegebenenfalls substituiert sein kann durch einen Rest ausgewählt aus der Gruppe F, Cl, Br, Methyl, Ethyl, OH, $CF_3$ und Methoxy; |
| $R^4$, $R^5$ und $R^6$ | gleich oder verschieden Wasserstoff, Methyl oder Ethyl, |
| X | $NH_2$, NH-( $C_1$-$C_6$-Alkyl), N($C_1$-$C_6$-Alkyl)$_2$, wobei die beiden $C_1$-$C_6$-Alkyl-Gruppen gleich oder verschieden sein können, NH-COH, NH-CO($C_1$-$C_6$-Alkyl) oder F; |
| A | $-(CH_2)_3-$, $-CH_2-CH_2-O-$, $-CH_2-O-CH_2-$, $-(CH_2)_4-$, -CH($C_1$-$C_6$-Alkyl)-O-$CH_2$-, $-(CH_2)_2-O-CH_2-$, $-(CH_2)_3-O-$, $-(CH_2)_5-$, $-CH_2-O-(CH_2)_3-$, $-(CH_2)_2-O-(CH_2)_2-$, $-(CH_2)_3-O-CH_2-$, $-(CH_2)_4-O-$, $-CH_2-O-CH_2-CH_2-O-$, |

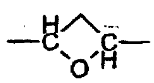 , 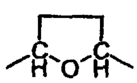 oder 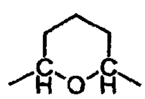

bedeuten können, gegebenenfalls in Form ihrer Racemate, ihrer Enantiomere, ihrer Diastereomere und ihrer Gemische, sowie gegebenenfalls ihrer pharmakologisch unbedenklichen Säureadditionssalze.

Bevorzugt sind die Verbindungen der allgemeinen Formel **1**
worin

| | |
|---|---|
| $R^1$ und $R^2$ | gleich oder verschieden Wasserstoff, Methyl, Ethyl, Methyloxy, Ethyloxy, OH, F, Cl oder Br; |
| $R^3$ und $R^{3'}$ | gleich oder verschieden Wasserstoff, F, Cl, Br, Methyl, Ethyl, OH, $CF_3$, Methoxy oder Phenyl, welches durch einen Rest ausgewählt aus der Gruppe F, Cl, Br und bevorzugt Methyl, substituiert ist; |
| $R^4$, $R^5$ und $R^6$ | gleich oder verschieden Wasserstoff oder Methyl; |
| X | $NH_2$, NH-(Methyl), N(Methyl)$_2$, NH-(Ethyl), N(Ethyl)$_2$, NH-COH, NH-COMe oder F; |
| A | $-CH_2-CH_2-O-$, $-CH_2-O-CH_2-$, -CH(Methyl)-O-$CH_2$-, -CH(Ethyl)-O-$CH_2$-, -CH(iso-Propyl)-O-$CH_2$-, $-(CH_2)_2-O-CH_2-$, $-(CH_2)_3-O-$, $-CH_2-O-(CH_2)_3-$, $-(CH_2)_2-O-(CH_2)_2-$, $-(CH_2)_3-O-CH_2-$, $-(CH_2)_4-O-$, $-CH_2-O-CH_2-CH_2-O-$, |

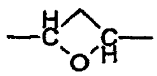 , 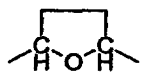 oder 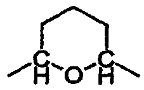

bedeuten können, gegebenenfalls in Form ihrer Racemate, ihrer Enantiomere, ihrer Diastereomere und ihrer Gemische, sowie gegebenenfalls ihrer pharmakologisch unbedenklichen Säureadditionssalze.

Besonders bevorzugt sind Verbindungen der allgemeinen Formel **1**
worin

| | |
|---|---|
| $R^1$ und $R^2$ | gleich oder verschieden Wasserstoff oder F; |
| $R^3$ und $R^{3'}$ | gleich oder verschieden Wasserstoff, F, Cl, Br, $CF_3$ oder Methyl; |
| $R^4$, $R^5$ und $R^6$ | gleich oder verschieden Wasserstoff oder Methyl; |
| X | $NH_2$, NH-(Methyl), $N(Methyl)_2$, NH-COH, NH-COMe oder F; |
| A | -CH(Methyl)-O-$CH_2$-, -$CH_2$-O-$CH_2$- oder |

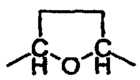

bedeuten können, gegebenenfalls in Form ihrer Racemate, ihrer Enantiomere, ihrer Diastereomere und ihrer Gemische, sowie gegebenenfalls ihrer pharmakologisch unbedenklichen Säureadditionssalze.

Erfindungsgemäß von besonderer Bedeutung sind Verbindungen der allgemeinen Formel **1**, worin

| | |
|---|---|
| $R^1$ und $R^2$ | gleich oder verschieden Wasserstoff oder F; |
| $R^3$ und $R^{3'}$ | gleich oder verschieden Wasserstoff, F, Cl, Br, $CF_3$ oder Methyl; |
| $R^4$, $R^5$ und $R^6$ | gleich oder verschieden Wasserstoff oder Methyl; |
| X | $NH_2$, NH-(Methyl) oder NH-COH; |
| A | -CH(Methyl)-O-$CH_2$-, -$CH_2$-O-$CH_2$- oder |

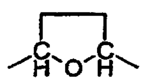

bedeuten können, gegebenenfalls in Form ihrer Racemate, ihrer Enantiomere, ihrer Diastereomere und ihrer Gemische, sowie gegebenenfalls ihrer pharmakologisch unbedenklichen Säureadditionssalze.

Erfindungsgemäß von gleichrangiger Bedeutung sind Verbindungen der allgemeinen Formel **1** worin

| | |
|---|---|
| $R^1$ und $R^2$ | gleich oder verschieden Wasserstoff oder F; |
| $R^3$ und $R^{3'}$ | Wasserstoff; |
| $R^4$, $R^5$ und $R^6$ | gleich oder verschieden Wasserstoff oder Methyl; |
| X | F; |
| A | -CH(Methyl)-O-$CH_2$-, |

bedeuten können, gegebenenfalls in Form ihrer Racemate, ihrer Enantiomere, ihrer Diastereomere und ihrer Gemische, sowie gegebenenfalls ihrer pharmakologisch unbedenklichen Säureadditionssalze.

In den vorstehend genannten Verbindungen der allgemeinen Formel **1** steht $R^1$, wenn von Wasserstoff verschieden, bevorzugt in ortho- oder para-Position, besonders bevorzugt in ortho-Position in Relation zur Brücke A. In den vorstehend genannten Verbindungen der allgemeinen Formel **1** steht $R^2$, wenn von Wasserstoff verschieden, bevorzugt in ortho-Position in Relation zur Brücke A.

In den vorstehend genannten Verbindungen der allgemeinen Formel **1** steht $R^3$, wenn von Wasserstoff verschieden, bevorzugt in 7-Position, also para in Relation zur Gruppe X. In den vorstehend genannten Verbindungen der allgemeinen Formel **1** steht $R^{3'}$, wenn von Wasserstoff verschieden, bevorzugt in 9-Position, also ortho in Relation zur Gruppe X.

Erfindungsgemäß besonders bevorzugt sind Verbindungen der Formel **1** in denen $R^{3'}$ Wasserstoff bedeutet.

Von besonderem Interesse sind Verbindungen der allgemeinen Formel **1** ausgewählt aus der Gruppe bestehend aus:

- (2R,6S,2S')-10-Amino-3-[2(2,6-difluorphenyl-methoxy)-propyl]-1,2,3,4,5,6-hexahydro-6,11,11-trimethyl-2,6-methano-3-benzazocin-dihydrochlorid;
- (2R,6S,2S')-10-Amino-3-[2-(benzyloxy)propyl]-1,2,3,4,5,6-hexahydro-6,11,11-trimethyl-2,6-methano-3-benzazocin-dihydrochlorid;
- (2R,6S,11R,2"S)-10-Amino-3-[2(2,6-difluorphenyl-methoxy)-propyl]-1,2,3,4,5,6-hexahydro-6,11-dimethyl-2,6-methano-3-benzazocin-dihydrochlorid;
- (2R,6S,11S,2"S)-10-Amino-3-[2(2,6-difluorphenyl-methoxy)-propyl]-1,2,3,4,5,6-hexahydro-6,11-dimethyl-2,6-methano-3-benzazocin-dihydrochlorid;
- (2R,6S)-10-Amino-3-[2(2,6-difluorphenyl-methoxy)-ethyl]-1,2,3,4,5,6-hexahydro-6,11,11-trimethyl-2,6-methano-3-benzazocin-dihydrochlorid;
- (2R,6S,2"S,5"S)-10-Amino-3-[5"-phenyl-tetrahydrofuran-2"-yl)methyl]-1,2,3,4,5,6-hexahydro-6,11,11-trimethyl-2,6-methano-3-benzazocin-dihydrochlorid;
- (2R,6S,2"S)-10-Acetamino-3-[2(benzyloxy)propyl]-1,2,3,4,5,6-hexahydro-6,11,11-trimethyl-2,6-methano-3-benzazocin-hydrochlorid;
- (2R,6S,2"S)-10-Acetamino-3-[2(2,6-difluorphenyl-methoxy)-propyl]-1,2,3,4,5,6-hexahydro-6,11,11-trimethyl-2,6-methano-3-benzazocin-hydrochlorid;
- (2R,6S,2"S)-10-Formylamino-3-[2(2,6-difluorphenyl-methoxy)-propyl]-1,2,3,4,5,6-hexahydro-6,11,11-trimethyl-2,6-methano-3-benzazocin-hydrochlorid;
- (2R,6S,2"S)-10-Methylamino-3-[2(2,6-difluorphenyl-methoxy)-propyl]-1,2,3,4,5,6-hexahydro-6,11,11-trimethyl-2,6-methano-3-benzazocin-dihydrochlorid;
- (2R,6S,2"S)-10-Dimethylamino-3-[2(2,6-difluorphenyl-methoxy)-propyl]-1,2,3,4,5,6-hexahydro-6,11,11-trimethyl-2,6-methano-3-benzazocindihydrochlorid;
- (2R,6S,2"S)-10-Ethylamino-3-[2(2,6-difluorphenyl-methoxy)-propyl]-1,2,3,4,5,6-hexahydro-6,11,11-trimethyl-2,6-methano-3-benzazocin-dihydrochlorid;
- (2R,6S,2"S)-10-Amino-7-methyl-3-[2-(benzyloxy)propyl]-1,2,3,4,5,6-hexahydro-6,11,11-trimethyl-2,6-methano-3-benzazocin-dihydrochlorid;
- (2R,6S,2"S)-10-Amino-7-methyl-3-[2-(2,6-difluorphenyl-methoxy)propyl]-1,2,3,4,5,6-hexahydro-6,11,11-trimethyl-2,6-methano-3-benzazocindihydrochlorid;
- (2R,6S,2"S)-10-Amino-7-chlor-3-[2(benzyloxy)-propyl]-1,2,3,4,5,6-hexahydro-6,11,11-trimethyl-2,6-methano-3-benzazocin-dihydrochlorid;
- (2R,6S,2"S)-10-Amino-7-chlor-3-[2(2,6-difluorphenyl-methoxy)-propyl]-1,2,3,4,5,6-hexahydro-6,11,11-trimethyl-2,6-methano-3-benzazocin-dihydrochlorid;
- (2R,6S,2"S)-10-Amino-7-brom-3-[2(benzyloxy)-propyl]-1,2,3,4,5,6-hexahydro-6,11,11-trimethyl-2,6-methano-3-benzazocin-dihydrochlorid;
- (2R,6S,2"S)-10-Amino-7-brom-3-[2(2,6-difluorphenyl-methoxy)-propyl]-1,2,3,4,5,6-hexahydro-6,11,11-trimethyl-2,6-methano-3-benzazocindihydrochlorid;
- (2R,6S,2"S)-10-Amino-7-fluor-3-[2-(benzyloxy)propyl]-1,2,3,4,5,6-hexahydro-6,11,11-trimethyl-2,6-methano-3-benzazocin-dihydrochlorid;
- (2R,6S,2"S,5"S)-10-Amino-7-methyl-3-[5"-phenyl-tetrahydrofuran-2"-yl)methyl]-1,2,3,4,5,6-hexahydro-6,11,11-trimethyl-2,6-methano-3-benzazocindihydrochlorid;
- (2R,6S,2"S)-10-Amino-7-trifluormethyl-3-[2(benzyloxy)-propyl]-1,2,3,4,5,6-hexahydro-6-methyl-2,6-methano-3-benzazocin-dihydrochlorid;
- (2R,6S,2"S)-10-Amino-7-(4-methylphenyl)-3-[2(2,6-difluorphenyl-methoxy)-propyl]-1,2,3,4,5,6-hexahydro-6-methyl-2,6-methano-3-benzazocin-dihydrochlorid;

$C_1$-$C_6$-Alkyl steht für einen unverzweigten oder verzweigten Kohlenwasserstoffrest mit 1 bis 6 Kohlenstoffatomen die gegebenenfalls auch Ringsysteme enthalten konnen. Die Alkylsubstituenten können gleich oder verschieden sein und gegebenenfalls mit einem oder mehreren Halogenatomen substituiert sein, vorzugsweise Fluor. Als Beispiele seien Methyl, Ethyl, Propyl, Isopropyl, Butyl, Isobutyl, Pentyl, Isopentyl, Neopentyl, Hexyl, Cylopropylmethyl, Cylobutyl, Cyclopentyl, Cyclohexyl genannt Gegebenenfalls werden für die vorstehend genannten Alkylgruppen auch gängige Abkürzungen wie beispielsweise Me für Methyl, Et für Ethyl, Prop für Propyl etc. verwendet.

$C_1$-$C_6$-Alkyloxy steht für einen unverzweigten oder verzweigten Kohlenwasserstoffrest mit 1 bis 6 Kohlenstoffatomen, der über ein Sauerstoffatom verknüpft ist. Die Alkyloxysubstituenten können gleich oder verschieden sein. Als Beispiele seien Methyloxy, Ethyloxy, Propyloxy, Isopropyloxy, Butyloxy, Isobutyloxy, Pentyloxy, Isopentyloxy, Neopentyloxy, Hexyloxy, Cylopropylmethyloxy, Cylobutyloxy, Cyclopentyloxy, Cyclohexyloxy genannt. . Gegebenenfalls werden für die vorstehend genannten Alkyloxygruppen auch die Bezeichungen Alkoxy verwendet. Dementsprechend kann statt Methyloxy auch Methoxy, Ethyloxy auch Ethoxy, Propyloxy auch Propoxy etc. als Bezeichnung Verwendung finden. Gegebenenfalls werden für die vorstehend genannten Alkyloxygruppen auch gängige Abkürzungen wie beispielsweise MeO für Methyloxy, EtO für Ethyloxy, PropO für Propyloxy etc. verwendet.

Die die Gruppe A repräsentierenden 2-bindigen Gruppen können erfindungsgemäß in beiden möglichen Ausrichtungen mit den sie benachbarenden Resten verknüpft sein.

Gegebenenfalls können die Verbindungen der allgemeinen Formel (**1**) in ihre Salze, insbesondere für die pharmazeutische Anwendung, in ihre pharmakologisch unbedenklichen Säureadditionssalze mit einer anorganischen oder organischen Säure, überführt werden. Als Säuren kommen hierfür beispielsweise Bemsteinsäure, Bromwasserstoffsäure, Essigsäure, Fumarsäure, Maleinsäure, Methansulfonsäure, Milchsäure, Phosphorsäure, Salzsäure, Schwefelsäure, Weinsäure oder Zitronensäure in Betracht. Femer können Mischungen der vorgenannten Säuren eingesetzt werden.

Die erfindungsgemäßen Verbindungen sind Blocker des spannungsabhängigen Natriumkanals. Es handelt sich dabei um Verbindungen, die Batrachotoxin (BTX) mit hoher Affinität ($K_i$ < 1000 nM) kompetitiv oder nicht-kompetitiv von der Bindungsstelle am Natriumkanal verdrängen. Solche Substanzen zeigen eine "usedependency" bei der Blokkade der Natriumkanäle, d.h. für die Bindung der Substanzen an dem Natriumkanal müssen die Natriumkanäle zunächst aktiviert werden. Die maximale Blockade der Natriumkanäle wird erst nach wiederholter Stimulation der Natriumkanäle erreicht. Demzufolge binden die Substanzen bevorzugt an Natriumkanäle, die vermehrt aktiviert werden.

Dadurch sind die Substanzen in der Lage, bevorzugt in den Körperregionen wirksam zu werden, die pathologisch überstimuliert sind. Darunter fallen Erkrankungen wie Arrhythmien, Spasmen, kardiale und Gehimischämien, Schmerzen sowie neurodegenerative Erkrankungen verschiedener Genese. Beispielsweise kann genannt werden: Epilepsie, Hypoglykämie, Hypoxie, Anoxie, Gehimtrauma, Gehimoedem, Gehirn-Schlaganfall, perinatale Asphyxie, Degenerationen des Cerebellums, amyotrope laterale Sklerose, Morbus Huntington, Morbus Alzheimer, Morbus Parkinson, Zyklophrenie, Hypotonie, Herzinfakt, Herzrhythmusstörungen, Angina pectoris, chronischer Schmerz, neuropathischer Schmerz sowie Lokalanaesthesie.

Ein weiterer Aspekt der Erfindung zielt folglich auf die Verwendung von Verbindungen der allgemeinen Formel **1,** als Arzneimittel, insbesondere als Arzneimittel, in denen die Blockade des spannungsabhängigen Natriumkanals einen therapeutischen Nutzen entfalten kann.
Bevorzugt ist die Verwendung der erfindungsgemäßen Verbindungen der allgemeinen Fomel **1** zur Herstellung eines Arzneimittels zur Vorbeugung oder Behandlung von Arrhythmien, Spasmen, kardialen und Gehimischämien, Schmerzen sowie neurodegenerative Erkrankungen.

Besonders bevorzugt ist die vorstehend genannte Verwendung der erfindungsgemäßen Verbindungen der allgemeinen Formel **1** zur Herstellung eines Arzneimittels zur Vorbeugung oder Behandlung von Epilepsie, Hypoglykämie, Hypoxie, Anoxie, Gehirntrauma, Gehimoedem, Gehirn-Schlaganfall, perinatale Asphyxie, Degenerationen des Cerebellums, amyotrope laterale Sklerose, Morbus Huntington, Morbus Alzheimer, Morbus Parkinson, Zyklophrenie, Hypotonie, Herzinfakt, Herzrhythmusstörungen, Angina pectoris, chronischer Schmerz, neuropathischer Schmerz sowie Lokalanaesthesie.

Als Testsystem für den Nachweis der Natriumkanal blockierenden Wirkung dient die BTX-Bindung am Natriumkanal [S.W. Postma & W.A. Catterall, Mol. Pharmacol 25, 219-227 (1984)] sowie patch-clamp Experimente, in denen gezeigt werden kann, daß die erfindungsgemäßen Verbindungen den elektrisch stimulierten Natriumkanal in einer "use-dependent" Art und Weise blockieren [W.A. Catterall, Trends Pharmacol. Sci., 8, 57-65(1987)]. Durch die Auswahl des Zellsystems (z.B. neuronale, kardiale, DRG Zellen) kann die Wirkung der Substanzen auf verschiedene Natriumkanalsubtypen untersucht werden.

Die Natriumkanal blockierende Eigenschaft der erfindungsgemäßen Verbindungen kann durch die Blockade des Veratridin induzierten Glutamat-releases nachgewiesen werden [S. Villauneva, P. Frenz, Y. Dragnic, F. Orrego, Brain Res. 461, 377-380 (1988)]. Veratridin ist ein Toxin, das den Natriumkanal permanent öffnet. Dadurch kommt es zu einem erhöhten Einstrom von Natriumionen in die Zelle. Über die oben beschriebene Kaskade führt dieser Natriumeinstrom in neuronalem Gewebe zu einer gesteigerten Freisetzung von Glutamat. Durch die erfindungsgemäßen Verbindungen läßt sich diese Glutamatfreisetzung antagonisieren.

Antikonvulsive Eigenschaften der erfindungsgemäßen Substanzen wurden durch eine protektive Wirkung gegen Krämpfe, die durch einen maximalen Elektroschock in Mäusen ausgelöst wurden, belegt [M. A. Rogawski & R. J. Porter, Pharmacol. Rev. 42, 223-286 (1990)].

Neuroprotektive Eigenschaften wurden durch protektive Wirkung in einem Ratten-MCAO-Modell [U. Pschom & A. J. Carter, J. Stroke Cerebrovascular Diseases, 6, 93-99 (1996)], einem Malonat induziertem Läsionsmodell [ M. F. Beal, Annals of Neurology, 38, 357-366 (1995) und J.B. Schulz, R.T. Matthews, D.R. Henshaw und M.F. Beal, Neuroscience, 71, 1043-1048 (1996)] sowie einem MPTP induzierten Degenerationsmodell [ J.P. Steiner, et al. Proc. Natl. Acad. Sci. 94, 2019-2024 (1997)] belegt.

Analgetische Wirkung wurde in einem Formalin induzierten Schmerzmodell [D. Dubuisson und S.G. Dennis, Pain, 4, 161-174 (1977)] sowie in einem Ligatur-Modell [G. J. Bennett & Y.-K. Xie, Pain 33, 87-107 (1988)] belegt.

Femer wurde beschrieben, daß Natriumkanalblocker zur Therapie der Zyklophrenie (manisch depressive Erkrankung) eingesetzt werden können [J. R. Calabrese, C. Bowden, M.J. Woyshville; in: Psychopharmacology: The

Fourth Generation of Progress (Eds.: D. E. Bloom and D. J. Kupfer) 1099-1111. New York: Raven Press Ltd.].

Die erfindungsgemäßen Verbindungen 1 lassen sich in Analogie zu an sich bekannten Synthesemethoden herstellen. Ein möglicher Syntheseweg ist in Schema 1 dargestellt.

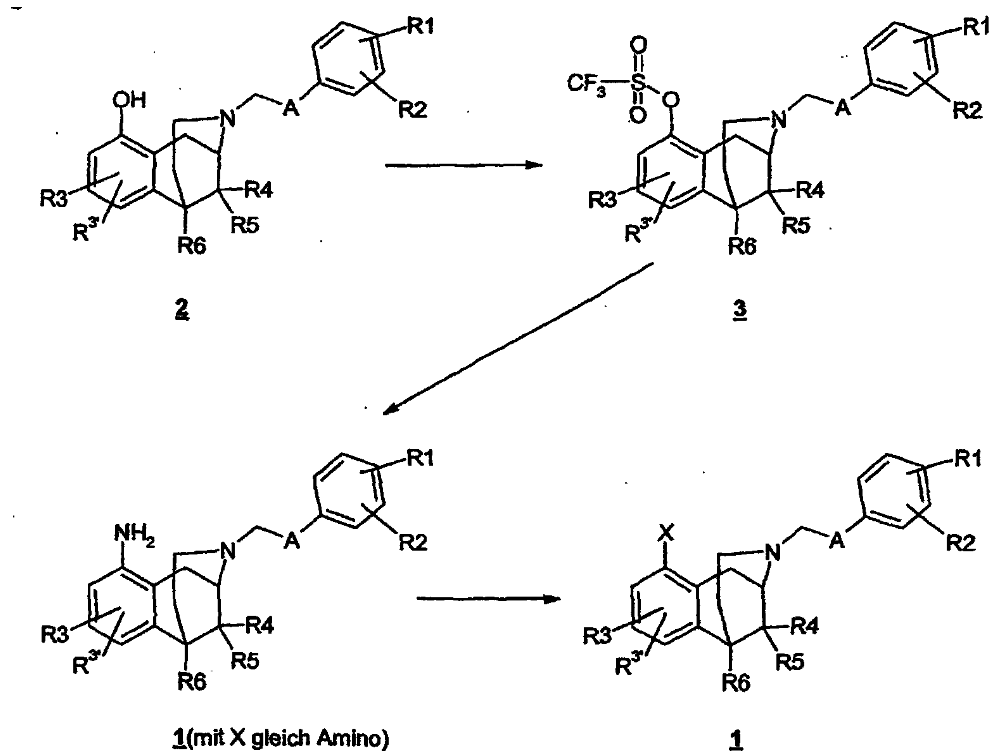

2

3

1(mit X gleich Amino)

1

Schema 1:

Schlüsselschritt ist die Überführung des Phenols 2 in die Verbindung der Formel 1, in der X = $NH_2$ bedeutet, die über eine Buchwald-Reaktion gelingt [J. P. Wolfe, J. Ahman, J. P. Sadighi, R. A. Singer, S. L. Buchwald, THL 1997, 6367-6370].

Die für diese Umsetzung benötigten Triflate 3 lassen sich ausgehend von den Verbindungen 2 mit Trifluormethansulfonsäureanhydrid in Gegenwart einer Hilfsbase darstellen. Als Hilfsbasen kommen erfindungsgemäß organische Amine wie beispielsweise Dimethylaminopyridin, Pyridin oder auch tert. Amine wie beispielsweise Trimethylamin, Triethylamin, Diisopropylethylamin oder DBU (Diazabicycloundecen) in Betracht. Von den vorstehend genannten Aminen ist die Verwendung der tert. Amine bevorzugt, besonders bevorzugt wird Triethylamin als Hilfsbase eingesetzt. Die Umsetzung wird in aprotischen, organischen Lösemitteln, bevorzugt in Lösemitteln ausgewählt aus der Gruppe bestehend aus Dimethylformamid, Dimethylacetamid, Methylenchlorid, Toluol oder auch Tetrahydrofuran durchgeführt. Als besonders bevorzugt wird an dieser Stelle Methylenchlorid genannt. Vorzugsweise werden die Umsetzungen zu den Triflaten 3 bei Temperaturen unterhalb der Raumtemperatur, besonders bevorzugt bei -50°C - 0°C, höchst bevorzugt zwischen -30°C und -5°C durchgeführt. Nach 0,5 bis 4 h wird bis zum vollständigen Umsatz bei Raumtemperatur weitergerührt (ca. 1-12 h). Nach der Aufarbeitung werden die so erhalten Rohprodukte 3 ohne weitergehende Reinigung in einem aromatischen organischen Lösemittel, vorzugsweise ausgewählt aus der Gruppe Toluol, Benzol oder Xylol, besonders bevorzugt Toluol, unter Palladium-Katalyse, vorzugsweise in Gegenwart eines Phosphin-Liganden, mit einer Stickstoffquelle, vorzugsweise mit Ketiminen, besonders bevorzugt mit Benzophenonimin, umgesetzt. Als Palladiumkatalysatoren kommen erfindungsgemäß in Betracht Tris(dibenzylidenaceton)-dipalladium, Palladium(II) acetat oder beispielsweise Tetrakistriphenylphosphin-Palladium. Als Phosphinliganden können beispielsweise Liganden ausgewählt aus der Gruppe DPPF, BINAP, p-tolBINAP, $PPh_2$-CHMe-P(tBu)$_2$, phosphin-substituierte Ferrocene oder auch Triphenylphosphin Verwendung finden. Bevorzugt wird als Katalysatorsystem Tris(dibenzylidenaceton)-dipalladium/BINAP eingesetzt. Die Reaktion wird vorzugsweise unter Feuchtigkeits- und Sauerstoffausschluß bei vorzugsweise erhöhter Temperatur geführt. Bevorzugt erfolgt die Umsetzung unter Rückfluß des eingesetzten Lösemittels. Das intermediär erhaltene Iminaddukt kann durch saure Hydrolyse, vorzugsweise mit verdünnten Mineralsäuren, besonders bevorzugt mit verdünnter Salzsäure in die erfindungsgemäßen Verbindungen 1 (mit X=$NH_2$) überführt werden. Die Reinigung der Produkte erfolgt durch Chromatographie an Kieselgel oder durch Kristallisation, bevorzugt durch

Kristallisation der pharmakologisch verträglichen Säureadditionssalze, beispielsweise der Hydrochloride.

Wie aus den vorstehend genannten Ausführungen ersichtlich, kommt den Triflaten der allgemeinen Formel 3 eine zentrale Bedeutung bei der Synthese der erfindungsgemäßen Verbindungen der allgemeinen Formel 1 zu.

Entsprechend zielt ein weiterer Aspekt der vorliegenden Erfindung auf die Zwichenverbindungen der allgemeinen Formel 3

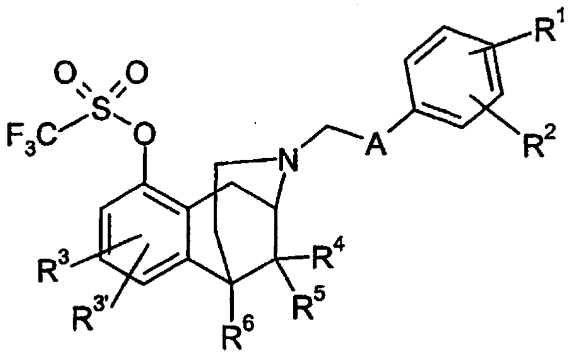

3

worin die Reste $R^1$, $R^2$, $R^3$, $R^{3'}$, $R^4$, $R^5$, $R^6$ und A die vorstehend angegebenen Bedeutungen aufweisen können.

Zur Synthese der erfindungsgemäßen Verbindungen der allgemeinen Formel 1 mit $X \neq NH_2$ kann wie nachfolgend beschrieben vorgegangen werden.

Verbindungen der Formel 1 in denen X = $NHC_1$-$C_6$-Alkyl oder $N(C_1$-$C_6$-Alkyl$)_2$ bedeutet, lassen sich nach an sich bekannten Verfahren durch Alkylierung von 1, in denen X = $NH_2$ bedeutet, durch reduktive Aminierung oder durch Acylierung gegebenenfalls in Gegenwart organischer Basen, mit anschließender Reduktion erhalten.

Zur Alkylierung kann wie folgt vorgegangen werden. Eine Verbindung der allgemeinen Formel 1 mit X=$NH_2$ wird in einem polaren organischen Lösemittel, wie Dimethylformamid, Dimethylactamid, Methylenchlorid, Tetrahydrofuran, bevorzugt Dimethylformamid oder Methylenchlorid gelöst. Die so erhaltene Lösung wird mit einer Base und einem entsprechenden Alkylierungsmittel versetzt Als Base kommen Alkali- und Erdalkalihydride, bevorzugt Natriumhydrid in Betracht Als Alkylierungsmittel können Alkylhalogenide, wie Alkylchlorid, Alkylbromid, besonders Alkyljodid sowie Alkyltosylate,- Mesylate, -Triflate, -Dialkylsulfate Verwendung finden. Nach üblicher Aufarbeitung können die alkylierten Verbindungen der allgemeinen Formel 1 durch Chromatographie an Kieselgel oder durch Kristallisation, gegebenenfalls in Form ihrer pharmakologisch verträglichen Säureaddtionssalze, bevorzugt als Hydrochloride, gereinigt werden.

Zur Darstellung der Verbindungen der allgemeinen Formel 1 über reduktive Aminierung werden unter ansonsten üblichen Reaktionsbedingungen unter Kühlung, vorzugsweise bei -50°C bis Raumtemperatur, besonders bevorzugt zwischen -30°C und 0°C die Amine 1 mit X=$NH_2$ in Gegenwart von Säuren wie verdünnter Salzsäure, verdünnter Essigsäure oder auch verdünnter Schwefelsäure mit Aldehyden oder Ketonen versetzt und die so intermediär gebildeten Schiffschen Basen oder Iminiumsalze anschließend reduziert. Die Reduktion erfolgt unter Verwendung von Metallhydriden wie beispielsweise Natriumborhydrid, $LiAlH_4$, Li-Alkoxyhydriden, $NaBH_4$, $NaBHCN_3$, $NaBH(OAc)_3$, bevorzugt gelangt Natriumborhydrid zur Anwendung. Nach üblicher Aufarbeitung können die alkylierten Verbindungen der allgemeinen Formel 1 durch Chromatographie an Kieselgel oder durch Kristallisation, gegebenenfalls in Form ihrer pharmakologisch verträglichen Säureaddtionssalze, bevorzugt als Hydrochloride, gereinigt werden.

Verbindungen der Formel 1 mit X = $NHCO(C_1$-$C_6$-Alkyl) lassen sich nach an sich bekannten Verfahren durch Acylierung von 1 mit X=$NH_2$, vorzugsweise mit Säurechloriden oder Anhydriden erhalten. Hierzu wird die Aminoverbindung der Formel 1 mit X=$NH_2$ in einem organischen Lösungsmittel suspendiert, mit einer organischen Base versetzt und das gewünschte Säurechlorid oder Anhydrid zugegeben. Die Mischung wird 40 bis 60 Minuten, bevorzugt 25 bis 45 Minuten bei Raumtemperatur gehalten. Geeignete organische Lösungsmittel sind Dimethylformamid, Dimethylacetamid, Methylenchlorid, Toluol oder auch Tetrahydrofuran, wobei Methylenchlorid bevorzugt ist. Geeignete organische Basen sind Dimethylaminopyridin, Pyridin, tert. Amine, beispielsweise Trimethylamin, Triethylamin, Diisopropylethylamin, DBU (Diazabicycloundecen). Nach Aufarbeitung werden die Produkte durch Chromatographie an Kieselgel oder durch Kristallisation, bevorzugt durch Kristallisation der pharmakologisch verträglichen Säureadditionssalze, beispielsweise der Hydrochloride gereinigt.

Ausgehend von den gemäß vorstehender Vorgehensweise erhältlichen Verbindungen der Formel 1 mit X = $NHCO(C_1$-$C_6$-Alkyl) lassen sich durch Reduktion mit Metallhydriden wie beispielsweise $LiAlH_4$, Li-Alkoxyhydriden, $NaBH_4$, $NaBHCN_3$, $NaBH(OAc)_3$, bevorzugt Natriumborhydrid, ebenfalls Verbindungen der Formel 1 mit z.B. X = NH ($C_1$-$C_6$-Alkyl) erhalten. Diese Umsetzungen werden vorzugsweise in etherischen organischen Lösemitteln, bevorzugt in Tetrahydrofuran oder Dioxan in Gegenwart von Lewissäuren, bevorzugt Bortrifluoridetherat, bei erhöhter Temperatur, bevorzugt oberhalb von 50°C, besonders bevorzugt unter Rückfluß des eingesetzten Lösemittels durchgeführt. Nach

Aufarbeitung werden die Produkte durch Chromatographie an Kieselgel oder durch Kristallisation, bevorzugt durch Kristallisation der pharmakologisch verträglichen Säureadditionssalze, beispielsweise der Hydrochloride gereinigt

Die formylierten Verbindungen der Formel **1** (X =NHCOH) lassen sich beispielsweise durch Umsetzung der Verbindungen der Formel **1** mit X=$NH_2$ mit Ameisensäure bei erhöhter Temperatur, vorzugsweise unter Rückfluß erhalten. Nach Aufarbeitung werden die Produkte durch Chromatographie an Kieselgel oder durch Kristallisation, bevorzugt durch Kristallisation der pharmakologisch verträglichen Säureadditionssalze, beispielsweise der Hydrochloride gereinigt.

Die Fluor-substituierten Verbindungen **1** ( mit X= F) lassen sich nach bekannten Verfahren durch Diazotierung von **1** mit X=$NH_2$ und anschließender Verkochung mit $BF_4^-$ erhalten. Vorzugsweise wird die Reaktion mit $NOBF_4$ als Diazotierungs- und Fluorierungsreagenz in etherischen Lösemitteln, vorzugsweise in Tetrahydrofuran oder Dioxan bei erhöhter Temperatur, bevorzugt oberhalb von 50°C, besonders bevorzugt unter Rückfluß des eingesetzten Lösemittels durchgeführt Nach Aufarbeitung werden die Produkte durch Chromatographie an Kieselgel oder durch Kristallisation, bevorzugt durch Kristallisation der pharmakologisch verträglichen Säureadditionssalze, beispielsweise der Hydrochloride gereinigt

Die in Schema 1 als Ausgangsverbindungen gekennzeichneten 1,2,3,4,5,6-Hexahydro-2,6-methano-3-benzazocin-10-ole (**2**) sind gemäß aus dem Stand der Technik bekannten Syntheseverfahren erhältlich. Diesbezüglich wird an dieser Stelle auf die Europäische Patentanmeldung EP 521422 sowie auf die Internationalen Patentanmeldungen WO 97/06146 und WO 99/14199 verwiesen.

Die Darstellung von Verbindungen der allgemeinen Formel **2**, in denen $R^3$ für Wasserstoff steht, gelingt ausgehend von den Verbindungen der allgemeinen Formel **4** in Analogie zu den in der WO99/14199 beschriebenen Synthesewegen. Zur Herstellung von Verbindungen der allgemeinen Formel **1**, in denen $R^3$ ungleich Wasserstoff ist, ist zuvor eine Modifikation der Verbindungen der allgemeinen Formel **4** erforderlich. Diese erfolgt unter analoger Anwendung prinzipiell bekannter Synthesevorschriften in der in den nachfolgenden Schemata beschriebenen Art und Weise.

Die Einführung einer p-Methylsubstitution ($R^3$ gleich Methyl) kann beispielsweise gemäß dem in Schema 2 angegebenen Weg erfolgen.

Zur Darstellung der bromierten Verbindung der Formel **5** werden die Verbindungen der Formel **4** in einem geeigneten Lösemittel, vorzugsweise in Essigsäure oder Trifluoressigsäure, besonders bevorzugt in einem Gemisch aus Essigsäure und Trifluoressigsäure gelöst und bei 0-23°C, bevorzugt bei 10°C portionsweise mit NBS (N-Bromsuccinimid) versetzt. Die Reaktion ist nach ca. 1 bis 5 Stunden, vollständig. Die Aufarbeitung erfolgt auf üblichem Wege. Die Reinigung der Produkte erfolgt je nach Kristallisationsneigung der Verbindungen **5** entweder durch Kristallisation oder durch Chromatographie.

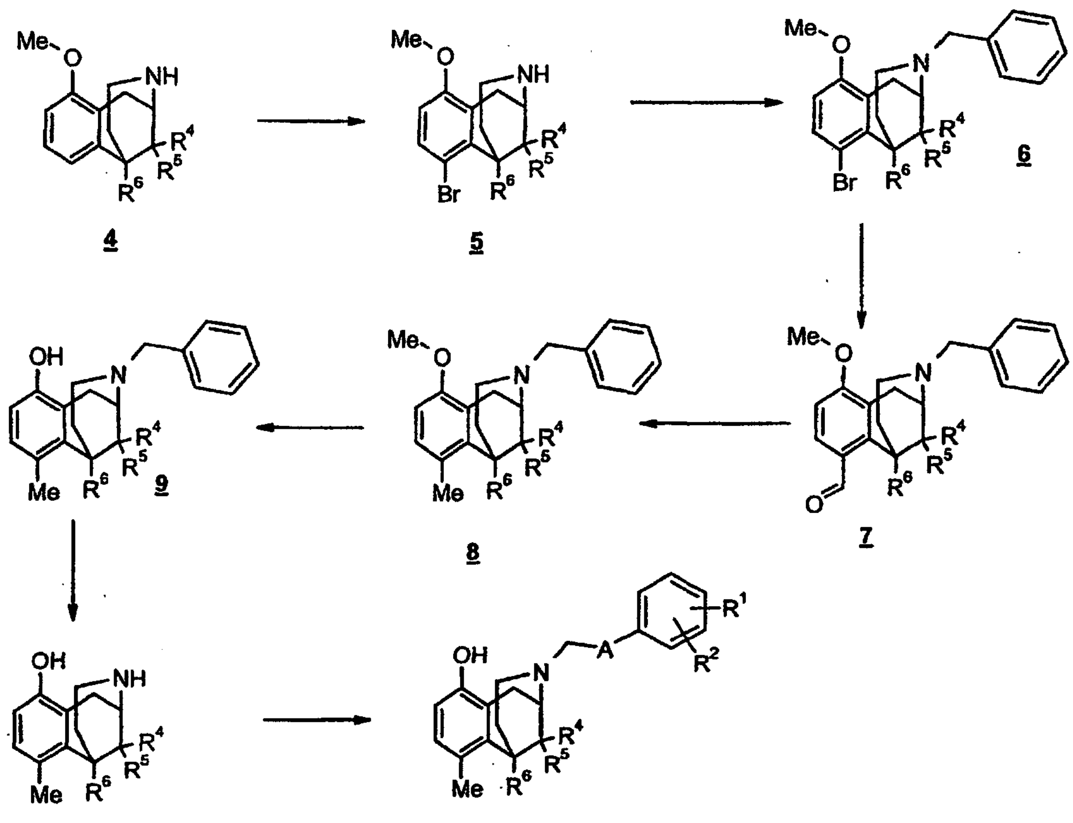

4 5 6 7 8 9 10

2 (mit $R^3$ gleich Methyl)
(mit $R^{3'}$ gleich Wasserstoff)

Schema 2:

Die Benzylierung der Verbindungen 5 zu den Verbindungen 6 wird vorzugsweise in einem organischen Lösemittel, bevorzugt in einem Lösemittel ausgewählt aus der Gruppe Dichlormethan, Dimethylformamid und Dimethylacetamid, besonders bevorzugt in Dimethylacetamid durchgeführt. Hierzu werden die Verbindungen 5 im Lösemittel gelöst und in Gegenwart einer Base bei einer Temperatur von 0-50°C, besonders bevorzugt bei Raumtemperatur (ca. 23°C) mit Benzylchlorid versetzt. Als Basen kommen organische oder anorganische Basen in Betracht. Als anorganische Base kommen die Alkali- oder die Erdalkalicarbonate des Lithiums, Natriums, Kaliums, Calziums wie Natriumcarbonat, Lithiumcarbonat, Kaliumcarbonat, Calziumcarbonat und bevorzugt Kaliumcarbonat in Betracht Als organische Base kommen bevorzugt organische Amine, besonders bevorzugt Diisopropylethylamin, Triethylamin, cyclische Amine wie DBU, oder Pyridin in Betracht Nach beendeter Zugabe des Benzylchlorids wird noch weitere 1 bis 6, bevorzugt 2 bis 4 Stunden bei erhöhter Temperatur, bevorzugt bei ca. 50-120°C, besonders bevorzugt bei ca. 100°C, maximal jedoch bei der Siedetemperatur des jeweiligen Lösemittels gerührt.

Die Aufarbeitung erfolgt auf üblichem Wege. Die Reinigung der Produkte erfolgt je nach Kristallisationsneigung der Verbindungen 6 entweder durch Kristallisation oder durch Chromatographie.

Die Darstellung der Verbindungen 7 gelingt aus den Verbindungen 6 durch Umsetzung mit geeigneten Formulierungsreagentien. Hierzu werden die Verbindungen 6 in einem geeigneten organischen Lösemittel, vorzugsweise in einem wasserfreien organischen Lösemittel, bevorzugt in einem etherischen Lösemittel ausgewählt aus der Gruppe Diethylether, Tetrahydrofuran und Dioxan, bevorzugt in Diethylether gelöst und bei niedriger Temperatur, bevorzugt bei unter -20°C, besonders bevorzugt bei einer Temperatur zwischen -50°C und -78°C mit Buthyllithium, vorzugsweise gelöst in n-Hexan versetzt. Nach ca. 0,5 bis 2 Stunden Rühren im vorstehend genannten Temperaturbereich, wird das Formulierungsreagenz, bevorzugt Dimethylformamid zugegeben und weitere 0,5 bis 2 Stunden, gegebenenfalls unter leicht erhöhter Temperatur, bevorzugt bei -30°C bis 0°C, besonders bevorzugt bei ca. -10°C gerührt. Die Aufarbeitung erfolgt auf üblichem Wege. Die Reinigung der Produkte erfolgt je nach Kristallisationsneigung der Verbindungen 7 entweder durch Kristallisation oder durch Chromatographie.

Die Reduktion der Verbindungen 7 führt zu den Methylverbindungen 8. Hierzu werden die Verbindungen 7 in

einem geeigneten organischen Lösemittel, vorzugsweise in einem wasserfreien organischen Lösemittel, bevorzugt in einem etherischen Lösemittel ausgewählt aus der Gruppe Diethylether, Tetrahydrofuran und Dioxan, bevorzugt in Tetrahydrofuran gelöst und mit einem geeigneten Reduktionsmittel, bevorzugt einem Metallsalzhydrid, besonders bevorzugt mit $NaBH_4$ oder $NaCNBH_3$, bevorzugt $NaCNBH_3$ versetzt. Ferner ist die Zugabe von Bortrifluorid, beziehungsweise $BF_3$-Etherat erforderlich. Nach Zugabe des Reduktionsmittels wird noch 1 bis 6 Stunden, bevorzugt 2 bis 4 Stunden bei konstanter Temperatur, vorzugsweise bei Raumtemperatur gerührt. Nach Zugabe eines protischen Lösemittels, besonders bevorzugt eines niederen Alkohols ausgewählt aus der Gruppe Methanol, Ethanol oder Isopropanol, besonders bevorzugt Methanol und Hydrolyse mit einer wässrigen Säure, bevorzugt mit wässriger Salzsäure wird weitere 0,5 bis 4 Stunden, gegebenenfalls bei erhöhter Temperatur, bevorzugt bei einer Temperatur oberhalb von 50°C, besonders bevorzugt unter Rückfluß des Lösemittels gerührt. Die Aufarbeitung erfolgt auf üblichem Wege. Die Reinigung der Produkte erfolgt je nach Kristallisationsneigung der Verbindungen **8** entweder durch Kristallisation oder durch Chromatographie.

Die Spaltung der Methoxygruppe in **8** führt zu den Hydroxyverbindungen **9** und gelingt unter Einwirkung starker Säuren, wie HI und HBr, besonders bevorzugt konzentrierter HBr bei erhöhter Temperatur, bevorzugt bei mehr als 50°C, besonders bevorzugt unter Rückfluß. Die Verwendung eines Lösemittels ist bei Einsatz von beispielsweise HBr nicht erforderlich. Die Reaktion ist nach ca. 1 bis 6, bevorzugt 2 bis 4 Stunden vollständig. Die Aufarbeitung erfolgt auf üblichem Wege. Die Reinigung der Produkte erfolgt je nach Kristallisationsneigung der Verbindungen **9** entweder durch Kristallisation oder durch Chromatographie. Die Spaltung der Methoxygruppe in **8** zu den Hydroxyverbindungen **9** kann alternativ mittels $BBr_3$ in Analogie zu der Vorgehensweise erfolgen, die für die Umsetzung der Verbindungen **17** zu den Hydroxyverbindungen **18** beschrieben wird.

Durch hydrogenolytische Abspaltung der Benzylgruppe lassen sich aus den Verbindungen **9** die Verbindungen **10** erhalten. Diese Reduktion erfolgt vorzugsweise durch katalytische Hydrierung, bevorzugt an Palladiumkatalysatoren oder an Raney-Nickel, besonders bevorzugt an Palladiumkatalysatoren in alkoholischen Lösemitteln, vorzugsweise in Methanol, bei Raumtemperatur. Die Aufarbeitung erfolgt auf üblichem Wege. Die Reinigung der Produkte erfolgt je nach Kristallisationsneigung durch Kristallisation oder mittels chromatographischer Methoden.

Die Darstellung der Verbindungen der allgemeinen Formel **2** gelingt ausgehend von den Verbindungen der allgemeinen Formel **10** in Analogie zu den in der WO99/14199 beschriebenen Synthesewegen.

Die Einführung einer p-Fluorsubstitution erfolgt nach dem im Schema 3 angegebenen Weg. Hierzu wird eine Verbindung der Formel 6 in einem geeigneten organischen Lösemittel, vorzugsweise in einem wasserfreien organischen Lösemittel, bevorzugt in einem etherischen Lösemittel ausgewählt aus der Gruppe Diethylether, Tetrahydrofuran und Dioxan, bevorzugt in Diethylether gelöst und bei niedriger Temperatur, bevorzugt bei unter -20°C, besonders bevorzugt bei einer Temperatur zwischen -50°C und -78°C mit Buthyllithium, vorzugsweise gelöst in n-Hexan versetzt. Nach ca. 0,5 bis 2 Stunden Rühren im vorstehend genannten Temperaturbereich, wird ein geeignetes Fluorierungsmittel, vorzugsweise N-Fluorbenzolsulfonimid gelöst in einem der vorstehend genannten Lösemittel langsam zugesetzt und weitere 0,5 bis 2 Stunden bei konstanter Temperatur gerührt. Die Aufarbeitung erfolgt auf üblichem Wege. Die Reinigung der Produkte erfolgt je nach Kristallisationsneigung durch Kristallisation oder mittels chromatographischer Methoden.

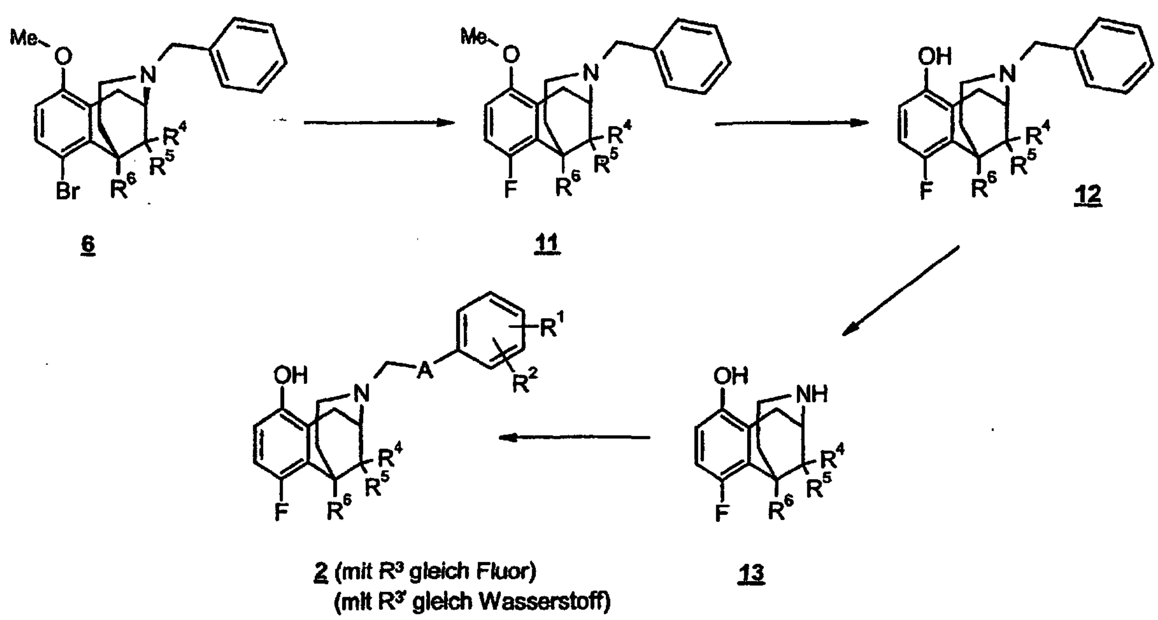

**6** **11** **12** **13**

**2** (mit $R^3$ gleich Fluor)
(mit $R^{3'}$ gleich Wasserstoff)

Schema 3:

Die Überführung der Verbindungen **11** in die Hydroxyverbindungen **12** erfolgt wie für die Umsetzung der Verbindungen **8** zu den Verbindungen **9** beschrieben. Die Überführung der Verbindungen **12** in die debenzylierten Verbindungen **13** erfolgt wie für die Umsetzung der Verbindungen **9** zu den Verbindungen **10** beschrieben.

Die Darstellung der Verbindungen der allgemeinen Formel **2** gelingt ausgehend von den Verbindungen der allgemeinen Formel **13** in Analogie zu den in der WO99/14199 beschriebenen Synthesewegen.

Die Einführung einer p-$CF_3$-Substitution erfolgt nach dem in Schema 4 dargestellten Syntheseweg. In einem ersten Schritt wird hierzu eine Verbindung der Formel **4** in ein Jodid der Formel **14** überführt. Diese Umsetzung erfolgt in Analogie zu der für die Umsetzung von **4** zu **5** beschriebenen Vorgehensweise unter Verwendung von NJS (N-Jodsuccinimid).

Aus den Verbindungen **14** lassen sich die N-geschützten Verbindungen **15** erhalten, in denen PG für eine Aminoschutzgruppe steht. Als Schutzgruppe kommen gängige zum Schutz der Aminofunktion gebräuchliche Gruppen in Betracht Beispielsweise seien genannt die Benzylgruppe, Benzyloxycarbonylgruppe und tert.-Butyloxycarbonylgruppe, wobei letztgenannte Gruppe bevorzugt ist. Zur Einführung der tert.-Butyloxycarbonylgruppe wird wie folgt vorgegangen. Die Umsetzung wird vorzugsweise in einem organischen Lösemittel, bevorzugt in einem Lösemittel ausgewählt aus der Gruppe Dichlormethan, Dimethylformamid und Dimethylacetamid, besonders bevorzugt in Dichlormethan durchgeführt. Hierzu werden die Verbindungen **14** im Lösemittel gelöst und in Gegenwart einer Base bei einer Temperatur von -50°C bis 10°C, bevorzugt bei -20°C bis 0°C gelöst und mit einer anorganischen Base versetzt. Als anorganische Base kommen die Alkali- oder die Erdalkalihydroxide des Lithiums, Natriums, Kaliums, Calziums wie Natriumhydroxid, Lithiumhydroxid, Kaliumhydroxid, Calziumhydroxid und bevorzugt Kaliumhydroxid, gegebenenfalls in wässriger Lösung in Betracht Anschließend wird Di-t-butyldicarbonat, gegebenenfalls gelöst in einem der vorstehend genannten organischen Lösemittel zugegeben. Nach beendeter Zugabe läßt man noch weitere 0,5 bis 6, bevorzugt 1 bis 3 Stunden, gegebenenfalls unter höherer Temperatur, vorzugsweise bei Raumtemperatur weiterrühren. Die Aufarbeitung erfolgt auf üblichem Wege. Die Reinigung der Produkte erfolgt je nach Kristallisationsneigung der Verbindungen **15** entweder durch Kristallisation oder durch Chromatographie.

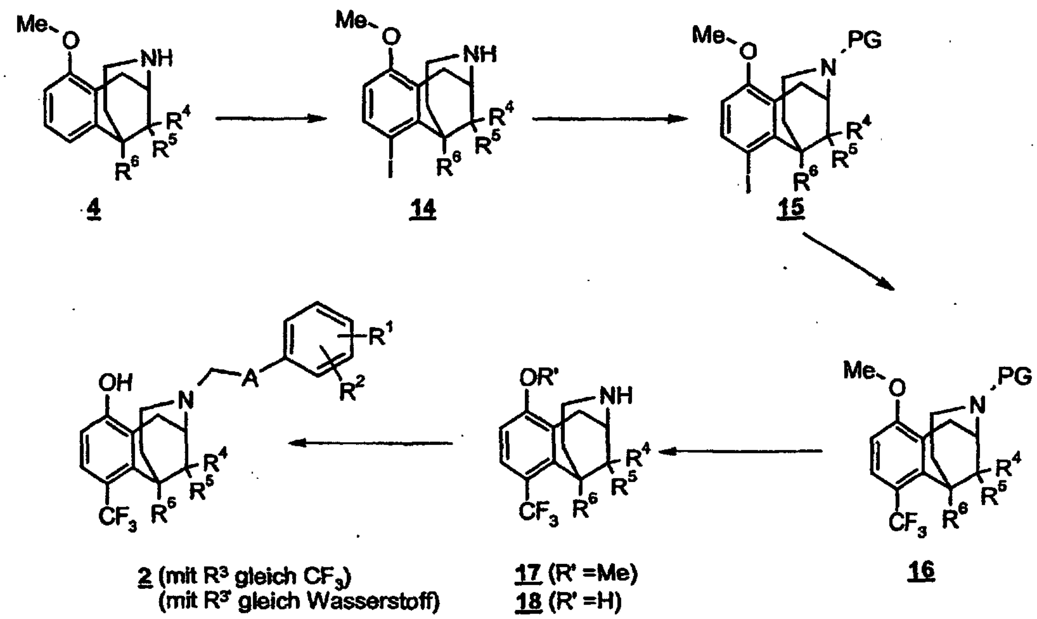
**4** **14** **15**

**2** (mit $R^3$ gleich $CF_3$) (mit $R^{3'}$ gleich Wasserstoff) **17** (R' =Me) **18** (R' =H) **16**

Schema 4:

Ausgehend von den Verbindungen **15** gelingt die Darstellung der Trifluormethyl-Verbindungen **16**. Hierzu wird eine Verbindung **15** in einem geeigneten organischen Lösemittel, bevorzugt in einem polaren organischen Lösemittel ausgewählt aus Dimethylacetamid, Dimethylsulfoxid oder Dimethylformamid, besonders bevorzugt Dimethylformamid gelöst und mit Fluorsulfonyl-difluoressigsäuremethylester und CuI, bevorzugt wasserfreies CuI, versetzt. Anschließend wird die Reaktionsmischung erwärmt auf eine Temperatur im Bereich von 50-90°C, besonders bevorzugt auf 70°C. Bei konstanter Temperatur wird zwischen 1 Tag und 4 Tagen, bevorzugt ca. 2 Tage gerührt. Je nach Reaktionsverlauf kann gegebenenfalls die weitere Zugabe von Fluorsulfonyl-difluoressigsäuremethylester und CuI erforderlich sein. Die

Aufarbeitung erfolgt auf üblichem Wege. Die Reinigung der Produkte erfolgt je nach Kristallisationsneigung der Verbindungen **16** entweder durch Kristallisation oder durch Chromatographie.

Die Abspaltung der Aminoschutzgruppe führt ausgehend von den Verbindungen **16** zu den Verbindungen **17**. Die Verfahren zur Abspaltung der einsetzbaren Schutzgruppen sind aus dem Stand der Technik bekannt An dieser Stelle sei exemplarisch lediglich eine mögliche Verfahrensvariante zur Abspaltung der tert.-Butyloxycarbonylschutzgruppe erwähnt. Diese gelingt durch Umsetzung von **16** mit Trifluoressigsäure in einem geeigneten organischen Lösemittel, vorzugsweise in Dichlormethan bei Raumtemperatur innerhalb eines Zeitraums von 1 bis 3 Stunden. Die Aufarbeitung erfolgt auf üblichem Wege. Die Reinigung der Produkte erfolgt je nach Kristallisationsneigung der Verbindungen **17** entweder durch Kristallisation oder durch Chromatographie.

Die Spaltung der Methoxygruppe führt ausgehend von den Verbindungen **17** zu den. Verbindungen **18** und gelingt durch Umsetzung mit $BBr_3$-Lösung in Gegenwart von Trifluoressigsäure in einem geeigneten organischen Lösemittel, vorzugsweise in Dichlormethan bei niedriger Temperatur, vorzugsweise bei unter -50°C, besonders bevorzugt zwischen -75°C und -60°C. Nach beendeter Zugabe der Reaktanden bei vorstehend genannter Temperatur wird die Reaktions durch Rühren bei einer Temperatur im Bereich von -20°C bis 20°C, bevorzugt bei ca. 0°C über einen Zeitraum von 4 bis 12 Stunden vervollständigt. Die Aufarbeitung erfolgt auf üblichem Wege. Die Reinigung der Produkte erfolgt je nach Kristallisationsneigung der Verbindungen **18** entweder durch Kristallisation oder durch Chromatographie. Alternativ zu der vorstehend beschriebenen Vorgehensweise kann die Spaltung der Methoxygruppe in **17** zu den Hydroxyverbindungen **18** auch unter Verwendung starker Säuren erfolgen. Hierzu sei auf die entsprechenden Ausführungen bezüglich der Umsetzung der Verbindungen **8** zu den Hydroxyverbindungen **9** verweisen.

Die Darstellung der Verbindungen der allgemeinen Formel **2** gelingt ausgehend von den Verbindungen der allgemeinen Formel **18** in Analogie zu den in der WO99/14199 beschriebenen Syntheswegen.

Die Einführung einer p-Phenylsubstitution erfolgt nach dem in Schema 5 dargestellten Weg.

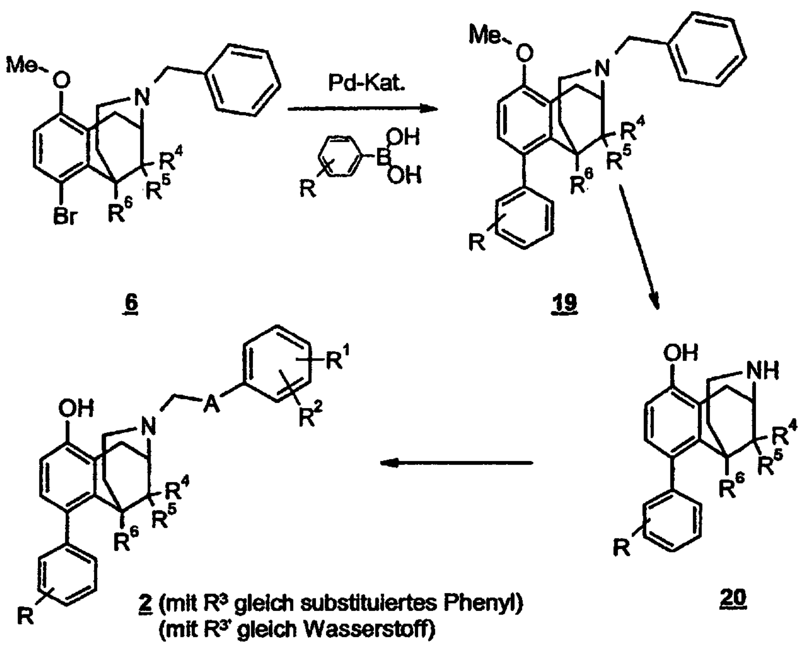


**6**

**19**

**20**

**2** (mit $R^3$ gleich substituiertes Phenyl) (mit $R^{3'}$ gleich Wasserstoff)

Schema 5:

Ausgehend von den Verbindungen **6** werden die Verbindungen **19** durch Umsetzung mit geeignet substituierten Boranaten und anschließender Transmetallierung mit Palladium(0)-Verbindungen erhalten. Als Boranate gelangen mit dem Rest R substituierte Phenylboronsäuren zur Anwendung. Zur Umsetzung wird **6** in einem geeigneten organischen Lösemittel, vorzugsweise in einem wasser- und suerstofffreien organischen Lösemittel, bevorzugt in einem etherischen Lösemittel ausgewählt aus der Gruppe Diethylether, Tetrahydrofuran und Dioxan, bevorzugt in Dioxan gelöst und nacheinander mit dem vorstehend genannten Boronsäurederivat, Kaliumphosphat und einem geeigneten Palladium(0)katalysator versetzt. Als Pd(0)-Katalysator kommen erfindungsgemäß in Betracht Tris(dibenzylidenaceton)-dipalladium, Palladium(II)acetat oder beispielsweise Tetrakistriphenylphosphin-Palladium bevorzugt Tris(dibenzylidenaceton)-paladium(0) in Betracht. Femer ist der Zusatz von Phosphinen oder Phosphiten erforderlich. Als Phosphinliganden können beispielsweise Liganden ausgewählt aus der Gruppe DPPF, BINAP, p-tolBINAP, $PPh_2$-CHMe-P

$(tBu)_2$, phosphin-substituierte Ferrocene, Triphenylphosphin oder Trimethylphosphit, bevorzugt Trimethylphosphit Verwendung finden. Nach Zugabe aller Reaktionskomponenten wird bei erhöhter Temperatur, bevorzugt bei einer Temperatur oberhalb von 50°C, besonders bevorzugt und Rückfluß des Lösemittels 0,5 bis 2 Stunden gerührt. Die Aufarbeitung erfolgt auf üblichem Wege. Die Reinigung der Produkte erfolgt je nach Kristallisationsneigung der Verbindungen **19** entweder durch Kristallisation oder durch Chromatographie.

Die Überführung der Verbindungen **19** in die Hydroxyverbindungen **20** erfolgt wie für die Umsetzung der Verbindungen **8** zu den Verbindungen **10** (über **9**) beschrieben.

Die Darstellung der Verbindungen der allgemeinen Formel **2** gelingt ausgehend von den Verbindungen der allgemeinen Formel **20** in Analogie zu den in der WO99/14199 beschriebenen Synthesewegen.

Halogensubsituierte Derivate lassen sich beispielsweise durch Umsetzung der erfindungsgemäßen Verbindungen **1**, in denen X für $NH_2$ und $R^3$ für Wasserstoff steht, mit NJS, NBS oder NCS erhalten (Schema 6). Dabei erhält man in der Regel Gemische der para-, ortho- und para-/ortho-disubstituierten Verbindungen, die chromatogaphisch getrennt werden können.

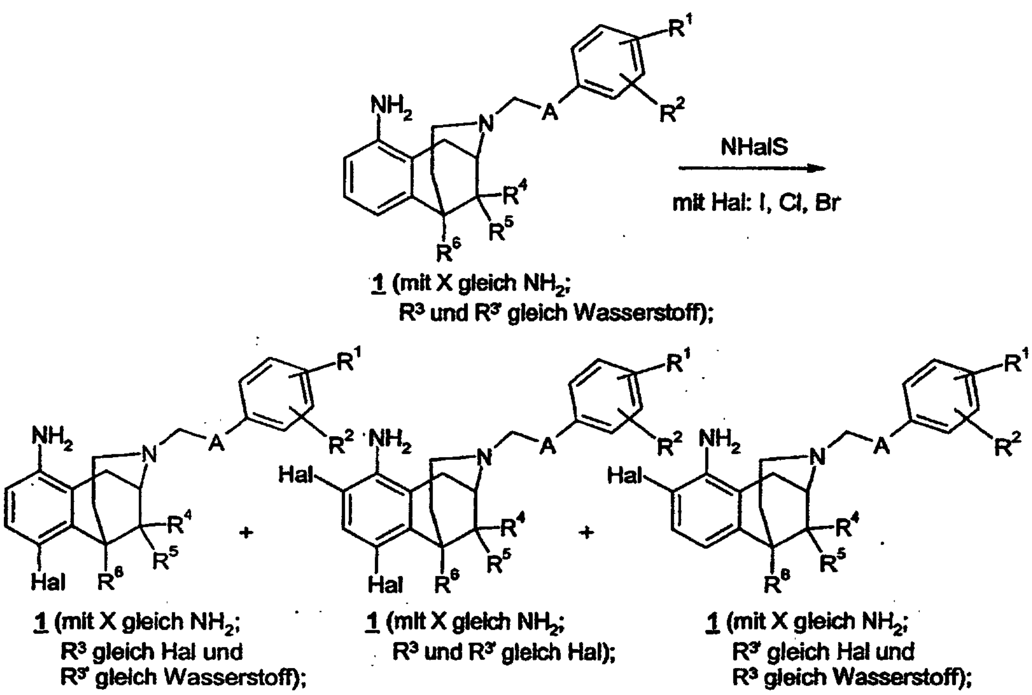


**1** (mit X gleich $NH_2$; $R^3$ und $R^{3'}$ gleich Wasserstoff);

**1** (mit X gleich $NH_2$; $R^3$ gleich Hal und $R^{3'}$ gleich Wasserstoff); **1** (mit X gleich $NH_2$; $R^3$ und $R^{3'}$ gleich Hal); **1** (mit X gleich $NH_2$; $R^{3'}$ gleich Hal und $R^3$ gleich Wasserstoff);

Schema 6:

Die Darstellung von Verbindungen der allgemeinen Formel **2**, in denen $R^3$ für Wasserstoff steht, gelingt ausgehend von den Verbindungen der allgemeinen Formel **4** in Analogie zu den in der WO99/14199 beschriebenen Synthesewegen. Zur Darstellung der Verbindungen **2**, in denen $R^3$ Wasserstoff bedeutet, wird von den Verbindungen der Formel **21** ausgegangen (Schema 7). Diese sind aus den Verbindungen **4** erhältlich. Die Verbindungen **21** sind in der WO99/14199 offenbart.

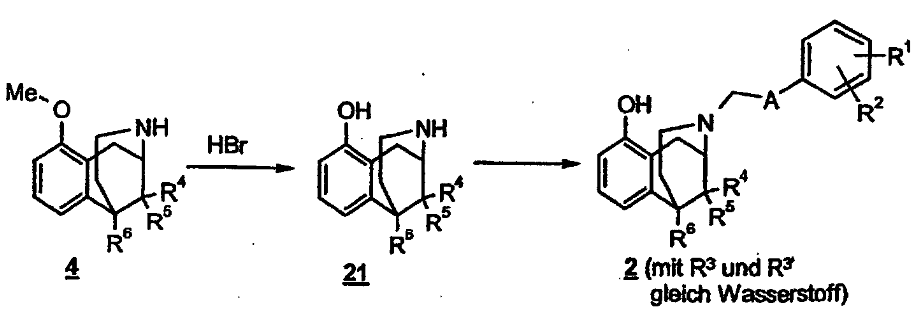


**4** **21** **2** (mit $R^3$ und $R^{3'}$ gleich Wasserstoff)

Schema 7:

Die nachfolgenden Beispiele dienen der näheren Erläuterung der Erfindung, ohne diese jedoch auf die beispielhaft offenbarten Verbindungen und Verfahren zu beschränken.

**I. Synthese der Vorstufen 4 bis 20:**

Die Verbindungen der Formel

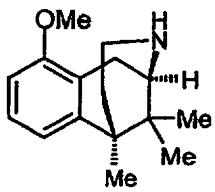
**4a**,
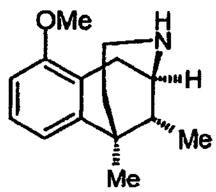
**4b**,
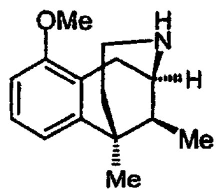
**4c** und
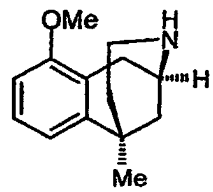
**4d**

sind beispielsweise aus der WO99114199 bekannt.

***Verbindungen der Formel 5:***

(2R,6S)-7-Brom-10-methoxy-1,2,3,4,5,6-hexahydro-6,11,11-trimethyl-2,6-methano-3-benzazocin-hydrochlorid **5a:**

24,7 g (0,1 Mol) ) (2R,6S)-10-Methoxy-1,2,3,4,5,6-hexahydro-6,11,11-trimethyl-2,6-methano-3-benzazocin (**4a**) werden in 220 mL Essigsäure und 22 mL Trifluoressigsäure gelöst, auf 10 °C gekühlt und portionsweise mit 19,6 g NBS (N-Bromsuccinimid) versetzt. Man rührt 3 h bei 10 °C, zieht das Lösungsmittel i. Vak. ab und versetzt den Rückstand mit eisgekühlter Ammoniaklösung. Anschließend wird zweimal mit je 200 mL Essigester extrahiert, die vereinigte organische Phase wird getrocknet und das Lösungsmittel i. Vak. abgezogen. Der Rückstand wird in Aceton gelöst und mit etherischer HCl das Hydrochlorid gefällt.
Ausbeute: 32,6 g (91%), Schmp.: 203 °C.

***Verbindungen der Formel 6:***

(2R,6S)-3-Benzyl-7-brom-10-methoxy-1,2,3,4,5,6-hexahydro-6,11,11-trimethyl-2,6-methano-3-benzazocin **6a:**

32,6 g (91 mmol) (2R,6S)-7-Brom-10-methoxy-1,2,3,4,5,6-hexahydro-6,11,11-trimethyl-2,6-methano-3-benzazocin-hydrochlorid **5a** werden in 160 mL Dimethylacetamid gelöst, mit 28 g $K_2CO_3$ und 18,9 g Benzylchlorid versetzt und 3h bei 100 °C gerührt. Anschließend wird das Lösungsmittel i. Vak. abgezogen, der Rückstand mit Wasser versetzt und zweimal mit je 200 mL Essigester extrahiert Die vereinigte organische Phase wird getrocknet, das Lösungsmittel i. Vak. abgezogen und der Rückstand an Kieselgel chromatographiert. Die geeigneten Fraktionen werden i. Vak. eingeengt. Ausbeute: 24,5 g (73%); Öl.

***Verbindungen der Formel 7:***

(2R,6S)-3-Benzyl-7-formyl-10-methoxy-1,2,3,4,5,6-hexahydro-6,11,11-trimethyl-2.6-methano-3-benzazocin **7a:**

24,5 g (59 mmol) (2R,6S)-3-Benzyl-7-brom-10-methoxy-1,2,3,4,5,6-hexahydro-6,11,11-trimethyl-2,6-methano-3-benzazocin **6a** werden in 250 mL abs. Ether gelöst, auf - 70 °C gekühlt und 40,7 mL BuLi (1,6 M in Hexan) zugetropft. Anschließend läßt man 1 h bei -60 °C rühren, tropft 10 mL DMF hinzu und läßt nach einer weiteren Stunde langsam auf -10 °C aufwärmen. Der Ansatz wird auf 500 mL Wasser gegeben und zweimal mit je 300 mL Ether extrahiert Die vereinigte organische Phase wird getrocknet, das Lösungsmittel i. Vak. abgezogen und der Rückstand an Kieselgel chromatographiert. Die geeigneten Fraktionen werden i. Vak. eingeengt. Ausbeute: 18,3 g (85%); Öl.

***Verbindungen der Formel 8:***

(2R,6S)-3-Benzyl-7-methyl-10-methoxy-1,2,3,4,5,6-hexahydro-6,11,11-trimethyl-2,6-methano-3-benzazocin **8a:**

8 g (22mmol) (2R,6S)-3-Benzyl-7-formyl-10-methoxy-1,2,3,4,5,6-hexahydro-6,11,11-trimethyl-2,6-methano-3-benzazocin **7a** werden in 80 mL THF gelöst, mit 4 g $NaCNBH_3$ und 20 mL $BF_3$-Etherat versetzt und 3 h bei RT gerührt. Anschließend werden vorsichtig 100 mL Methanol zugetropft, 100 mL 2 N HCl zugegeben und 1 h unter Rückfluß gekocht. Das Lösungsmittel wird i. Vak. abgezogen, der Rückstand mit Ammoniak versetzt und zweimal mit je 100 mL Essigester extrahiert. Die vereinigte organische Phase wird getrocknet, das Lösungsmittel i. Vak. abgezogen und der Rückstand an Kieselgel chromatographiert. Die geeigneten Fraktionen werden i. Vak. eingeengt. Ausbeute: 5,5 g (72%); Öl.

***Verbindungen der Formel 9:***

(2R,6S)-3-Benzyl-7-methyl-10-hydroxy-1,2,3,4,5,6-hexahydro-6,11,11-trimethyl-2,6-methano-3-benzazocin **9a:**

5,3 g (15 mmol) (2R,6S)-3-Benzyl-7-methyl-10-methoxy-1,2,3,4,5,6-hexahydro-6,11,11-trimethyl-2,6-methano-3-benzazocin **8a** werden in 54 mL konz. HBr gelöst und 2 h unter Rückfluß gekocht. Der Ansatz wird i. Vak. eingeengt, der Rückstand mit Eis und konz. Ammoniak versetzt und zweimal mit je 50 mL Essigester extrahiert. Die vereinigte organische Phase wird getrocknet und das Lösungsmittel i. Vak. abgezogen. Der Rückstand wird in Aceton gelöst und mit etherischer HCl das Hydrochlorid gefällt. Ausbeute: 3,7 g (66%),

***Verbindungen der Formel 10:***

(2R,6S)-3-Benzyl-7-methyl-10-hydroxy-1,2,3,4,5,6-hexahydro-6,11,11-trimethyl-2.6-methano-3-benzazocin **10a:**

3,6 g (10 mmol) (2R,6S)-3-Benzyl-10-hydroxy-7-methyl-1,2,3,4,5,6-hexahydro-6,11,11-trimethyl-2,6-methano-3-benzazocin **9a** werden an 0,4 g PdC in 60 mL Methanol bei 5 bar 4 h hydriert. Anschließend wird filtriert und das Lösungsmittel i. Vak. abgezogen. Ausbeute 2.6 g (92%); Öl.

***Verbindungen der Formel 11:***

(2R,6S)-3-Benzyl-7-fluor-10-methoxy-1,2,3,4,5,6-hexahydro-6,11,11-trimethyl-2,6-methano-3-benzazocin **11a:**

8 g (20mmol) (2R,6S)-3-Benzyl-7-brom-10-methoxy-1,2,3,4,5,6-hexahydro-6,11,11-trimethyl-2,6-methano-3-benzazocin **6a** werden in 100 mL Ether gelöst, auf -65 °C gekühlt und unter Stickstoff 15 mL BuLi (1,6 M in Hexan) zugetropft. Nach 1 h werden 8 g N-Fluorbenzolsulfonimid in 100 mL THF zugetropft und 1 h bei -60 °C gerührt. Anschließend wird auf RT erwärmt, von unlöslichen Bestandteilen abgesaugt und das Lösungsmittel i.Vak. abgezogen. Der Rückstand wird in 100 mL Wasser aufgenommen, mit 10 mL konz. Ammoniak versetzt und zweimal mit je 150 mL Ether extrahiert. Die vereinigte organische Phase wird getrocknet, das Lösungsmittel i. Vak. abgezogen und der Rückstand an Kieselgel chromatographiert. Die geeigneten Fraktionen werden i. Vak. eingeengt. Ausbeute: 3,4 g (48%); Öl.

***Verbindungen der Formel 12:***

(2R,6S)-3-Benzyl-7-fluor-10-hydroxy-1,2,3,4,5,6-hexahydro-6,11,11-trimethyl-2,6-methano-3-benzazocin **12a:**

Herstellung analog zur Herstellung der Verbindung **9** ausgehend von **11**.Öl;

***Verbindungen der Formel 13:***

(2R,6S)-7-Fluor-10-hydroxy-1,2,3,4,5,6-hexahydro-6,11,11-trimethyl-2,6-methano-3-benzazocin **13a:**

Herstellung analog zur Herstellung der Verbindung **10** ausgehend von **12**;Öl;

***Verbindungen der Formel 14:***

(2R,6S)-7-Iod-10-hydroxy-1,2,3,4,5,6-hexahydro-6,11,11-trimethyl-2,6-methano-3-benzazocin **14a:**

32,5 g (0,13 Mol) ) (2R,6S)-10-Methoxy-1,2,3,4,5,6-hexahydro-6,11,11-trimethyl-2,6-methano-3-benzazocin **4a** werden in 330 mL Essigsäure und 110 mL Trifluoressigsäure gelöst, auf 5 °C gekühlt und portionsweise mit 32,9 g NJS versetzt. Man rührt 3 h bei 10 °C, zieht das Lösungsmittel i. Vak. ab und versetzt den Rückstand mit eisgekühlter Ammoniaklösung. Anschließend wird zweimal mit je 200 mL Essigester extrahiert, die vereinigte organische Phase wird getrocknet und das Lösungsmittel i. Vak. abgezogen. Der Rückstand wird in Essigester gelöst und mit etherischer HCl das Hydrochlorid gefällt. Ausbeute: 31,7 g (59%), Schmp.: 231 °C.

***Verbindungen der Formel 15:***

(2R,6S)-3-t-Butyloxycarbonyl-7-iod-10-methoxy-1,2,3,4,5,6-hexahydro-6,11,11-trimethyl-2,6-methano-3-benzazocin **15a:**

31,6 g (78 mmol) (2R,6S)-7-Iod-10-methoxy-1,2,3,4,5,6-hexahydro-6,11,11-trimethyl-2,6-methano-3-benzazocin-hydrochlorid **14a** werden in 350 mL Dichlormethan suspendiert, auf -5 °C gekühlt, mit 9 g KOH in 18 mL Wasser versetzt und 17,7 g Di-t-butyldicarbonat in 50 mL Dichlormethan zugetropft. Anschließend läßt man langsam auf RT kommen, rührt 2 h bei RT und versetzt mit 200 mL Wasser. Die organische Phase wird abgetrennt, gerocknel und i. Vak. das Lösungsmittel abgezogen. Ausbeute: 36,7 g (100%) zähes Öl.

***Verbindungen der Formel 16:***

(2R,6S)-3-t-Butyloxycarbonyl-7-trifluormethyl-10-methoxy-1,2,3,4,5,6-hexahydro-6,11,11-trimethyl-2,6-methano-3-benzazocin **16a:**

15,8 g (34 mmol) (2R,6S)-3-t-Butyloxycarbonyl-7-iod-10-methoxy-1,2,3,4,5,6-hexahydro-6,11,11-trimethyl-2,6-methano-3-benzazocin **15a** wird in 120 mL DMF gelöst, 10 g Fluorsulfonyl-difluoressigsäuremethylester und 2,3 g CuI zugegeben und auf 70 °C erwärmt. Nach 24 h werden weitere 3 g Fluorsulfonyl-difluoressigsäuremethylester und 0,7 g CuI zugegeben und nochmals 18 h bei 70 °C gerührt. Der Ansatz wurde abgekühlt, mit 500 mL Ether versetzt und von unlöslichen Bestandteilen abgesaugt. Das Filtrat wurde zweimal mit verd. Ammoniaklösung und mit Wasser gewaschen. Die organische Phase wurde getrocknet und das Lösungsmittel i. Vak abgezogen. Ausbeute13,6 g (98%); oranges Harz.

***Verbindungen der Formel 17:***

(2R,6S)-7-Trifluormethyl-10-methoxy-1,2,3,4,5,6-hexahydro-6,11,11-trimethyl-2,6-methano-3-benzazocin **17a:**

13,6 g (33 mmol) (2R,6S)-3-t-Butyloxycarbonyl-7-trifluormethyl-10-methoxy-1,2,3,4,5,6-hexahydro-6,11,11-trimethyl-2,6-methano-3-benzazocin **16a** werden in 250 mL Dichlormethan gelöst, 25 mL Trifluoressigsäure zugetropft und 2 h bei RT gerührt. Anschließend wird der Ansatz auf Eiswasser und Ammoniaklösung gegeben, die organische Phase abgetrennt, gerocknet und i. Vak. das Lösungsmittel abgezogen. Ausbeute: 10,7 g (100%) oranges Harz.

***Verbindungen der Formel 18:***

(2R,6S)-7-trifluormethyl-10-hydroxy-1,2,3,4,5,6-hexahydro-6,11,11-trimethyl-2,6-methano-3-benzazocin **18a:**

1,0 g (3,2 mmol) (2R,6S)-7-Trifluormethyl-10-methoxy-1,2,3,4,5,6-hexahydro-6,11,11-trimethyl-2,6-methano-3-benzazocin **17a** werden in 10 mL Dichlormethan gelöst, auf -65 °C gekühlt und 7 mL $BBr_3$-Lösung (1M in Dichlormethan) zugetropft. Anschließend läßt man auf 0 °C erwärmen und rührt 8 h bei dieser Temperatur. Unter Eiskühlung wird mit 10 mL MeOH versetzt und das Lösungsmittel i. Vak. abgezogen. Ausbeute: 1,2 g (100%), braunes Harz.

***Verbindungen der Formel 19:***

(2R,6S)-3-Benzyl-7-(4-methyl)phenyl-10-methoxy-1,2,3,4,5,6-hexahydro-6,11,11-trimethyl-2.6-methano-3-benzazocin **19a:**

4,1 g (10 mmol) (2R,6S)-3-Benzyl-7-brom-10-methoxy-1,2,3,4,5,6-hexahydro-6,11,11-trimethyl-2,6-methano-3-benzazocin **6a** werden in 100 mL entgasten Dioxan unter $N_2$ gelöst, mit 12,6 g $K_2PO_4$, 2,9 g 4-Methylphenylboronsäure, 0,92 g Tris(dibenzylidenaceton)-paladium(0) und 0,25 g Trimethylphosphit versetzt und 1 h unter Rückfluß gekocht. Anschließend läßt man abkühlen, saugt von den Salzen ab und zieht das Lösungsmittel i. Vak. ab. Der Rückstand wird an Kieselgel chromatographiert. Die geeigneten Fraktionen werden i. Vak. eingeengt.
Ausbeute: 3,8 g (89%); Öl.

***Verbindungen der Formel 20:***

(2R,6S)-10-Hydroxy-7-(4-methyl)phenyl-1,2,3,4,5,6-hexahydro-6,11,11-trimethyl-2,6-methano-3-benzazocin-hydrobromid **20a:**

Herstellung ausgehend von **19a** in Analogie zur Darstellung von **10a;**
Schmp.: >250 °C.

***Verbindungen der Formel 21:***

(2R,6S)-10-Hydroxy-1,2,3,4,5,6-hexahydro-6,11,11-trimethyl-2,6-methano-3-benzazocin-hydrobromid **21a:**

Herstellung ausgehend von **4a** in Analogie zur Herstellung von **9a**;

(2R,6S,11R)-10-Hydroxy-1,2,3,4,5,6-hexahydro-6,11-dimethyl-2,6-methano-3-benzazocin-hydrobromid **21b:**

Herstellung ausgehend von **4b** in Analogie zur Herstellung von **9a;**

(2R,6S,11S)-10-Hydroxy-1,2,3,4,5,6-hexahydro-6,11-dimethyl-2,6-methano-3-benzazocin-hydrobromid **21c:**

Herstellung ausgehend von **4c** in Analogie zur Herstellung von **9a;**

(2R,6S)-10-Hydroxy-1,2,3,4,5,6-hexahydro-6-methyl-2,6-methano-3-benzazocinhydrochlorid **21d:**

Herstellung ausgehend von **4d** in Analogie zur Herstellung von **9c;**

**II. Synthese der Verbindungen der Formel 2:**

(2R,6S,2"S)-10-Hydroxy-3-[2-(benzyloxy)propyl]-1,2,3,4,5,6-hexahydro-6,11,11-trimethyl-2,6-methano-3-benzazocin-dihydrochlorid **2a:**

1,6 g (6,9 mmol) (2R,6S)-10-Hydroxy-1,2,3,4,5,6-hexahydro-6,11,11-trimethyl-2,6-methano-3-benzazocin-hydrobromid **21a** werden in 30 mL Dichlormethan suspendiert und mit 6 mL N-Methylmorpholin versetzt. Nach 30 min. kühlt man auf -5°C und tropft langsam eine Lösung von 2,3 g (11,6 mmol) (-)-S-2-Benzyloxypropionsäurechlorid in 20 mL Dichlormethan hinzu. Man läßt noch 30 min. bei -5 °C rühren, versetzt mit 40 mL 2 N Salzsäure und trennt die organische Phase ab. Die organische Phase wird über $MgSO_4$ getrocknet, das Lösungsmittel im Vakuum abgezogen und der Rückstand in 80 mL THF aufgenommen. Zu dieser Lösung werden 1,0 g (26 mmol) $LiAlH_4$ hinzugegeben wobei die Temperatur auf 35 °C anstieg. Man läßt 30 min nachreagieren, versetzt mit 0,8 mL Wasser und 0,4 mL 5 N Natronlauge und trennt von anorganischen Niederschlag ab. Der Niederschlag wird mit 100 mL THF gewaschen und die vereinigten organischen Phasen im Vakuum eingeengt. Der Rückstand wird in 200 mL Ether aufgenommen, über $MgSO_4$ getrocknet und mit etherischer Salzsäure das Hydrochlorid gefällt. Die Kristalle werden abgetrennt und mit Aceton gewaschen.
Ausbeute: 2,1 g (73%), Schmp.: 254 °C, $[\alpha]_D^{25}$ = (-) 20,7° (C=1 in Methanol);

Unter Verwendung der entsprechenden Carbonsäuren wurden ferner die folgenden Verbindungen **2** erhalten:

(2R,6S,2S')-10-Hydroxy-3-[2(2,6-difluorphenyl-methoxy)-propyl]-1,2,3,4,5,6-hexahydro-6,11,11-trimethyl-2,6-methano-3-benzazocin-dihydrochlorid **2b**

Herstellung ausgehend von **21a** in Analogie zur Herstellung von **2a;**

(2R,6S,11R,2S')-10-Hydroxy-3-[2(2,6-difluorphenyl-methoxy)-propyl]-1,2,3,4,5,6-hexahydro-6,11-dimethyl-2,6-methano-3-benzazocin-dihydrochlorid **2c**

Herstellung ausgehend von **21b** in Analogie zur Herstellung von **2a;**

(2R,6S,11S,2S')-10-Hydroxy-3-[2(benzyloxy)-propyl]-1,2,3,4,5,6-hexahydro-6,11-dimethyl-2,6-methano-3-benzazocin-dihydrochlorid **2d**

Herstellung ausgehend von **21c** in Analogie zur Herstellung von **2a;**

(2R,6S,2S')-10-Hydroxy-3-[2(benzyloxy)-propyl]-1,2,3,4,5,6-hexahydro-6-methyl-2,6-methano-3-benzazocin-dihydrochlorid **2e**

Herstellung ausgehend von **21d** in Analogie zur Herstellung von **2a;**

(2R,6S)-10-Hydroxy-3-[2(2,6-difluorphenyl-methoxy)-ethyl]-1,2,3,4,5,6-hexahydro-6,11,11-trimethyl-2,6-methano-3-benzazocin-dihydrochlorid **2f**

Herstellung ausgehend von **21a** in Analogie zur Herstellung von **2a;**

(2R,6S,2S')-10-Hydroxy-3-[2(2,6-difluorphenyl-methoxy)-isopentyl]-1..2.3.4.5.6-hexahydro-6,11,11-trimethyl-2,6-methano-3-benzazocin-dihydrochlorid **2g**

Herstellung ausgehend von **21a** in Analogie zur Herstellung von **2a;**

(2R,6S)-10-Hydroxy-3-[2(benzyloxy)-ethyl]-1,2,3,4,5,6-hexahydro-6,11,11-trimethyl-2,6-methano-3-benzazocin-dihydrochlorid **2h**

Herstellung ausgehend von **21a** in Analogie zur Herstellung von **2a;**

(2R,6S)-10-Hydroxy-3-[2(2-fluorphenyl-methoxy)-ethyl]-1,2,3,4,5,6-hexahydro-6,11,11-trimethyl-2.6-methano-3-benzazocin-dihydrochlorid **2i**

Herstellung ausgehend von **21a** in Analogie zur Herstellung von **2a;**

(2R,6S)-10-Hydroxy-3-[2(2,4-difluorphenyl-methoxy)-ethyl]-1,2,3,4,5,6-hexahydro-6,11,11-trimethyl-2,6-methano-3-benzazocin-dihydrochlorid **2j**

Herstellung ausgehend von **21a** in Analogie zur Herstellung von **2a;**

(2R,6S)-10-Hydroxy-3-[2(2,6-dimethylphenyl-methoxy)-ethyl]-1,2,3,4,5,6-hexahydro-6,11,11-trimethyl-2,6-methano-3-benzazocin-dihydrochlorid **2k**

Herstellung ausgehend von **21a** in Analogie zur Herstellung von **2a;**

(2R,6S,2S')-10-Hydroxy-3-[2(benzyloxy)-butyl]-1,2,3,4,5,6-hexahydro-6,11,11-trimethyl-2,6-methano-3-benzazocin-dihydrochlorid **2l**

Herstellung ausgehend von **21a** in Analogie zur Herstellung von **2a;**

(2R,6S,2S')-10-Hydroxy-3-[2(2,6-difluorphenyl-methoxy)-butyl]-1,2,3,4,5,6-hexahydro-6,11,11-trimethyl-2,6-methano-3-benzazocin-dihydrochlorid **2m**

Herstellung ausgehend von **21a** in Analogie zur Herstellung von **2a;**

(2R,6S,2S')-10-Hydroxy-3-[2(2,6-difluorphenyl-methoxy)-propyl]-1,2,3,4,5,6-hexahydro-6-methyl-2,6-methano-3-benzazocin-dihydrochlorid **2n**

Herstellung ausgehend von **21d** in Analogie zur Herstellung von **2a;**

(2R,6S,2"R,5"S)-10-Hydroxy-3-[5"-phenyl-tetrahydrofuran-2"-yl)methyl]-1,2,3,4,5,6-hexahydro-6,11,11-trimethyl-2,6-methano-3-benzazocin-dihydrochlorid **2o**:

Herstellung ausgehend von **21a** in Analogie zur Herstellung von **2a;**

(2R,6S,2"S,5"S)-10-Hydroxy-3-[5"-phenyl-tetrahydrofuran-2"-yl)methyl]-1,2,3,4,5,6-hexahydro-6,11,11-trimethyl-2,6-methano-3-benzazocin-dihydrochlorid **2p:**

Herstellung ausgehend von **21a** in Analogie zur Herstellung von **2a;**

(2R,6S)-10-Hydroxy-3-[3(2,6-difluorphenyloxy)-propyl]-1,2,3,4,5,6-hexahydro-6,11,11-trimethyl-2,6-methano-3-benzazocin-dihydrochlorid **2q**

Herstellung ausgehend von **21a** in Analogie zur Herstellung von **2a;**

(2R,6S)-10-Hydroxy-3-[4(2,6-difluorphenyl)-butyl]-1,2,3,4,5,6-hexahydro-6,11,11-trimethyl-2,6-methano-3-benzazocin-dihydrochlorid **2r**

Herstellung ausgehend von **21a** in Analogie zur Herstellung von **2a;**

(2R,6S,2S')-10-Hydroxy-7-methyl-3-[2(2,6-difluorphenyl-methoxy)-propyl]-1,2,3,4,5,6-hexahydro-6,11,11-trimethyl-2,6-methano-3-benzazocin-dihydrochlorid **2s**

Herstellung ausgehend von **10a** in Analogie zur Herstellung von **2a;**

(2R,6S,2S')-10-Hydroxy-7-methyl-3-[2(benzyloxy)-propyl]-1,2,3,4,5,6-hexahydro-6,11,11-trimethyl-2,6-methano-3-benzazocin-dihydrochlorid **2t**

Herstellung ausgehend von **10a** in Analogie zur Herstellung von **2a;**

(2R,6S,2"S,5"S)-10-Hydroxy-7-methyl-3-[5"-phenyl-tetrahydrofuran-2"-yl)methyl]-1,2,3,4,5,6-hexahydro-6,11,11-trimethyl-2,6-methano-3-benzazocin-dihydrochlorid

**2u:** Herstellung ausgehend von **10a** in Analogie zur Herstellung von **2a;**

(2R,6S,2S')-10-Hydroxy-7-fluor-3-[2(benzyloxy)-propyl]-1,2,3,4,5,6-hexahydro-6,11,11-trimethyl-2.6-methano-3-benzazocin-dihydrochlorid **2v**

Herstellung ausgehend von **13a** in Analogie zur Herstellung von **2a;**

(2R,6S,2S')-10-Hydroxy-7-trifluormethyl-3-[2(benzyloxy)-propyl]-1,2,3,4,5,6-hexahydro-6,11,11-trimethyl-2,6-methano-3-benzazocin-dihydrochlorid **2w**

Herstellung ausgehend von **18a** in Analogie zur Herstellung von **2a;**

(2R,6S,2S')-10-Hydroxy-7-(4-methylphenyl)-3-[2(2,6-difluorphenyl-methoxy)-propyl]-1,2,3,4,5,6-hexahydro-6,11,11-trimethyl-2,6-methano-3-benzazocin-dihydrochlorid **2x**

Herstellung ausgehend von **20a** in Analogie zur Herstellung von **2a;**

(2R,6S)-10-Hydroxy-7-methyl-3-[2(2,6-difluorphenyl-methoxy)-ethyl]-1,2,3,4,5,6-hexahydro-6,11,11-trimethyl-2,6-methano-3-benzazocin-dihydrochlorid **2v**

Herstellung ausgehend von **10a** in Analogie zur Herstellung von **2a;**

**III. Synthese der erfindungsgemäßen Verbindungen der Formel 1:**

**Beispiel 1:** (2R,6S,2S')-10-Amino-3-[2(2,6-difluorphenyl-methoxy)-propyl]-1,2,3,4,5,6-hexahydro-6,11,11-trimethyl-2,6-methano-3-benzazocin-dihydrochlorid

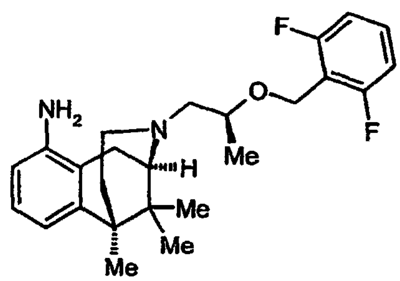

13,5 g ( 0.03 mol) (2R,6S,2"S)- 3-[2(2,6-Difluorphenyl-methoxy)-propyl]-1,2,3,4,5,6-hexahydro-6,11,11-trimethyl-2,6-methano-3-benzazocin-10-ol **2d** werden in 135 mL Dichlormethan gelöst, mit 30 mL Triethylamin und einer Spatelspitze 4-Dimethylaminopyridin versetzt und auf -10 °C gekühlt. Anschließend werden 6,8 mL Trifluormethansulfonsäureanhydrid zugetropft, 1h bei - 10 °C und weitere 2 h bei RT gerührt. Die Reaktionsmischung wird auf Eis gegeben, mit 50 mL Ammoniak versetzt, die organische Phase abgetrennt und einmal mit 50 mL Wasser gewaschen. Die organische Phase wird getrocknet, das Lösungsmittel i. Vak. abgezogen, der Rückstand in 350 mL Toluol aufgenommen und mit 21,6 g $CsCO_3$ und 10,8 g Benzophenonimin versetzt. Anschließend wird 30 min. Stickstoff durch die Suspension geleitet, mit 1,2 g BINAP und 0,6 g Tris(dibenzylidenaceton)-dipalladium versetzt und 6 h unter Rückfluß erhitzt. Nach Abkühlen wird mit 200 mL Wasser gewaschen, die organische Phase getrocknet und das Lösungsmittel i. Vak. abgezogen. Der Rückstand wird in Ethanol aufgenommen und mit etherischer HCl das Hydrochlorid gefällt. Ausbeute: 13,7 g (88%), Schmp.: 266 °C, $[\alpha]_D^{20}$ = (-) 37,0° (c=1 in MeOH).
Ferner wurden u.a. die folgenden Verbindungen erhalten:

**Beispiel 2:** (2R,6S,2"S)-10-Amino-3-[2-(benzyloxy)propyl]-1,2,3,4,5,6-hexahydro-6,11,11-trimethyl-2,6-methano-3-benzazocin-dihydrochlorid:

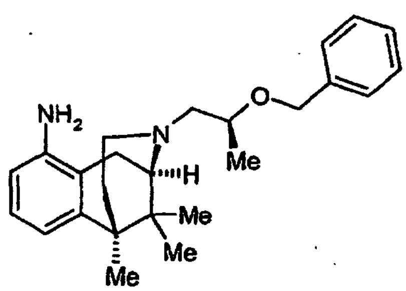

Herstellung ausgehend von Verbindung **2a** in Analogie zu Beispiel 1;

Schmp.: 257 °C, $[\alpha]_D^{20}$ = (-) 29,3° (c=1 in MeOH).

**Beispiel 3:** (2R,6S,11R,2"S)-10-Amino-3-[2(2,6-difluorphenyl-methoxy)-propyl]-1,2,3,4,5,6-hexahydro-6,11-dimethyl-2,6-methano-3-benzazocin-dihydrochlorid:

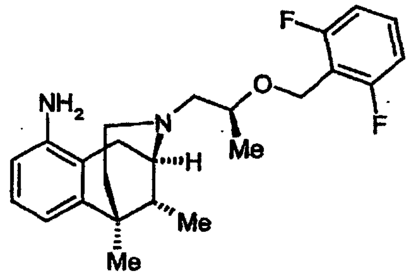

Herstellung ausgehend von Verbindung **2c** in Analogie zu Beispiel 1; Schmp.: 232 °C, $[\alpha]_D^{20}$ = (-) 4,1° (c=1 in MeOH).

**Beispiel 4:** (2R,6S,11S,2"S)-10-Amino-3-[2(2,6-difluorphenyl-methoxy)-propyl]-1,2,3,4,5,6-hexahydro-6,11-dimethyl-2,6-methano-3-benzazocin-dihydrochlorid:

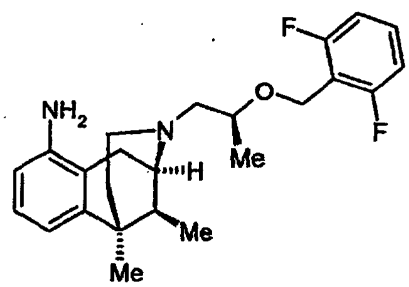

Herstellung ausgehend von Verbindung **2d** in Analogie zu Beispiel 1;
Schmp.: 267 °C, $[\alpha]_D^{20}$ = (-) 2,4° (c=1 in MeOH).

**Beispiel 5:** (2R,6S,2"S)-10-Amino-3-[2(2,6-difluorphenyl-methoxy)-propyl]-1,2,3,4,5,6-hexahydro-6-methyl-2,6-methano-3-benzazocin-dihydrochlorid:

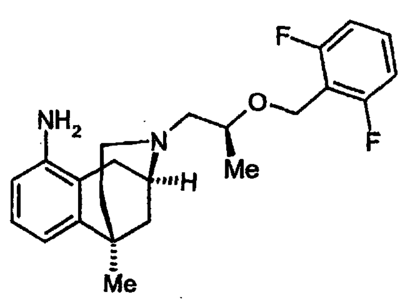

Herstellung ausgehend von Verbindung **2n** in Analogie zu Beispiel 1;
Schmp.: 187 °C, $[\alpha]_D^{20}$ = 19,7° (c=1 in MeOH).

**Beispiel 6:** (2R,6S,2S')-10-Amino-3-[2(2,6-difluorphenyl-methoxy)-butyl]-1,2,3,4,5,6-hexahydro-6,11,11-trimethyl-2,6-methano-3-benzazocin-dihydrochlorid

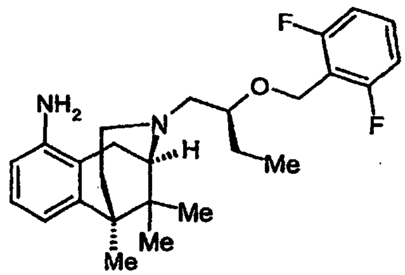

Herstellung ausgehend von Verbindung **2m** in Analogie zu Beispiel 1;
Schmp.: 200°C.

**Beispiel 7:** (2R,6S,2S')-10-Amino-3-[2(2,6-difluorphenyl-methoxy)-isopentyl]-1,2,3,4,5,6-hexahydro-6,11,11-trimethyl-2,6-methano-3-benzazocin-dihydrochlorid

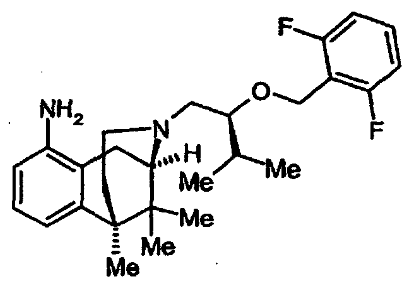

Herstellung ausgehend von Verbindung **2g** in Analogie zu Beispiel 1;
Schmp.: 274 °C, $[\alpha]_D^{20}$ = (-) 21,8° (c=1 in MeOH).

**Beispiel 8:** (2R,6S)-10-Amino-3-[2(2,6-difluorphenyl-methoxy)-ethyl]-1,2,3,4,5,6-hexahydro-6,11,11-trimethyl-2,6-methano-3-benzazocin-dihydrochlorid

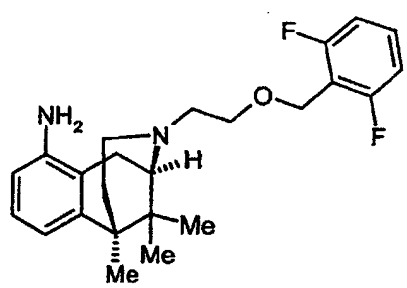

Herstellung ausgehend von Verbindung **2f** in Analogie zu Beispiel 1;
Schmp.: 270 °C, $[\alpha]_D^{20}$ = (-) 77,4° (c=1 in MeOH).

**Beispiel 9:** (2R,6S,2S')-10-Amino-3-[2(benzyloxy)-butyl]-1,2,3,4,5,6-hexahydro-6,11,11-trimethyl-2,6-methano-3-benzazocin-dihydrochlorid

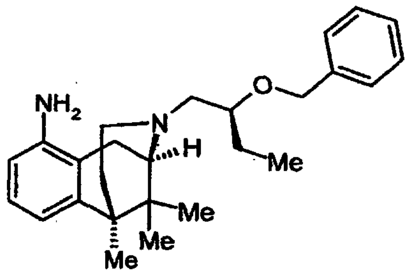

Herstellung ausgehend von Verbindung **2l** in Analogie zu Beispiel 1;
Schmp.: >190 °C(Zers.);.

**Beispiel 10:** (2R,6S)-10-Amino-3-[2(benzyloxy)-ethyl]-1,2,3,4,5,6-hexahydro-6,11,11-trimethyl-2,6-methano-3-benzazocin-dihydrochlorid

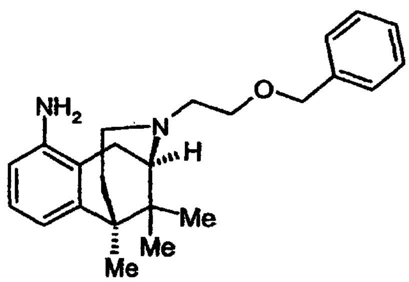

Herstellung ausgehend von Verbindung **2h** in Analogie zu Beispiel 1;
Schmp.: 220 °C;.

**Beispiel 11:** (2R,6S)-10-Amino-3-[2(2-fluorphenyl-methoxy)-ethyl]-1,2,3,4,5,6-hexahydro-6,11,11-trimethyl-2,6-methano-3-benzazocin-dihydrochlorid

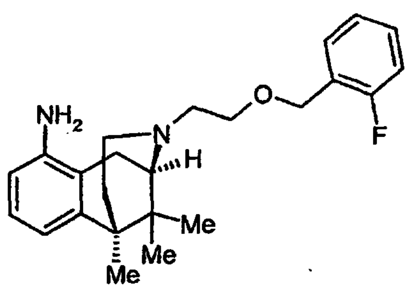

Herstellung ausgehend von Verbindung **2i** in Analogie zu Beispiel 1;
Schmp.: 220 °C;.

**Beispiel 12:** (2R,6S)-10-Amino-3-[3(2,6-difluorphenyloxy)-propyl]-1,2,3,4,5,6-hexahydro-6,11,11-trimethyl-2,6-methano-3-benzazocin-dihydrochlorid

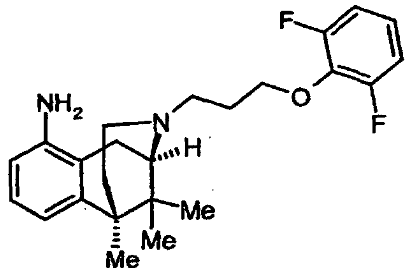

Herstellung ausgehend von Verbindung **2q** in Analogie zu Beispiel 1; Schmp.: 250 °C;.

**Beispiel 13:** (2R,6S)-10-Amino-3-[4(2,6-difluorphenyl)-butyl]-1,2,3,4,5,6-hexahydro-6,11,11-trimethyl-2,6-methano-3-benzazocin-dihydrochlorid

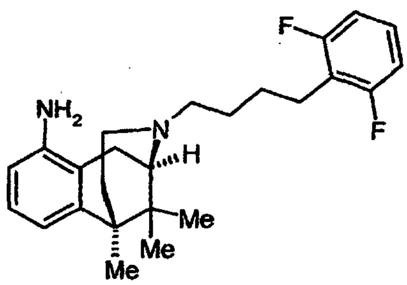

Herstellung ausgehend von Verbindung **2r** in Analogie zu Beispiel **1;** Schmp.: 250 °C;.

**Beispiel 14:** (2R,6S)-10-Amino-3-[2(2,4-difluorphenyl-methoxy)-ethyl]-1,2,3,4,5,6-hexahydro-6,11,11-trimethyl-2,6-methano-3-benzazocin-dihydrochlorid

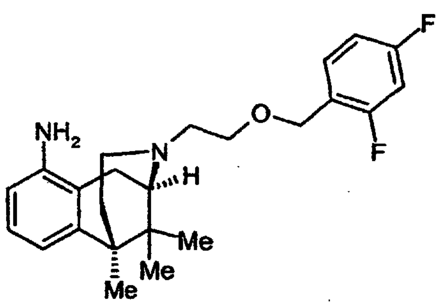

Herstellung ausgehend von Verbindung **2j** in Analogie zu Beispiel 1; Schmp.: 220 °C;.

**Beispiel 15:** (2R,6S)-10-Amino-3-[2(2,6-dimethylphenyl-methoxy)-ethyl]-1,2,3,4,5,6 hexahydro-6,11,11-trimethyl-2,6-methano-3-benzazocin-dihydrochlorid

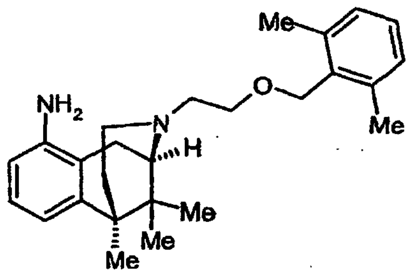

Herstellung ausgehend von Verbindung **2k** in Analogie zu Beispiel 1; Schmp.: 245 °C;.

**Beispiel 16:** (2R,6S,2"S,5"S)-10-Amino-3-[5"-phenyl-tetrahydrofuran-2"-yl)methyl]-1,2,3,4,5,6-hexahydro-6,11,11-trimethyl-2,6-methano-3-benzazocin-dihydrochlorid:

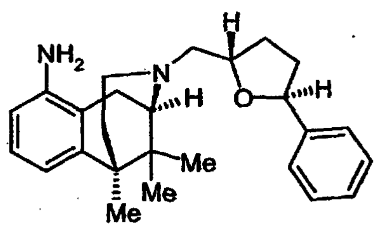

Herstellung ausgehend von Verbindung **2p** in Analogie zu Beispiel 1; Schmp.: 253 °C, $[\alpha]_D^{20}$ = (-) 135,4° (c=1 in MeOH).

**Beispiel 17:** (2R,6S,2"R,5"S)-10-Amino-3-[5"-phenyl-tetrahydrofuran-2"-yl)methyl]-1,2,3,4,5,6-hexahydro-6,11,11-trimethyl-2,6-methano-3-benzazocin-dihydrochlorid:

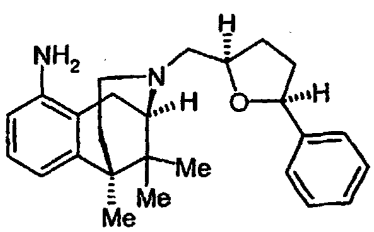

Herstellung ausgehend von Verbindung **2o** in Analogie zu Beispiel 1; Schmp.: 255 °C;.

**Beispiel 18:** (2R,6S,2"S)-10-Acetamino-3-[2(2,6-benzyloxy)propyl]-1,2,3,4,5,6-hexahydro-6,11,11-trimethyl-2,6-methano-3-benzazocin-hydrochlorid:

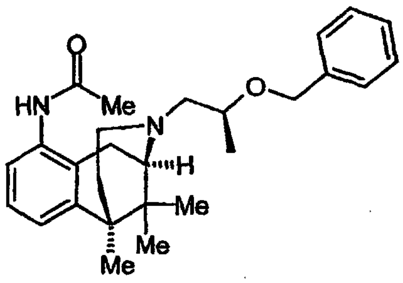

Zu 1,1 g ( 2,4 mmol) (2R,6S,2"S)-10-Amino-3-[2-(benzyloxy)propyl]-1,2,3,4,5,6-hexahydro-6,11,11-trimethyl-2,6-methano-3-benzazocin-dihydrochlorid (Beispiel 2) und 5 mL Triethylamin in 50 mL Dichlormethan werden 2 mL Acetanhydrid gegeben und 0,5 h bei RT gerührt. Anschließend wird i. Vak. eingeengt, mit 100 mL Ether versetzt und zweimal mit je 50 mL Wasser gewaschen. Die organische Phase wird getrocknet, das Lösungsmittel i. Vak. abgezogen, der Rückstand wird in Ether aufgenommen und mit etherischer HCl das Hydrochlorid gefällt Ausbeute: 0,8 g (73%), Schmp.: >100 °C, $[\alpha]_D^{20}$ = (-) 56,5° (c=1 in MeOH).

**Beispiel 19:** (2R,6S,2"S)-10-Acetamino-3-[2(2,6-difluorphenyl-methoxy)-propyl]-1,2,3,4,5,6-hexahydro-6,11,11-trimethyl-2,6-methano-3-benzazocin-hydrochlorid:

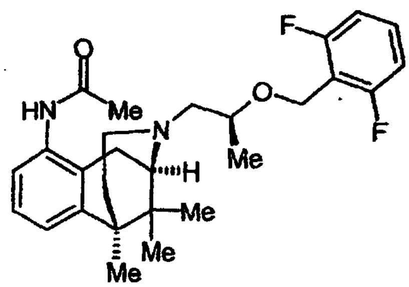

Herstellung ausgehend von Beispiel 1 in Analogie zu Beispiel 18;
Schmp.: 125 °C, $[\alpha]_D^{20}$ = (-) 58,7° (c=1 in MeOH).

**Beispiel 20:** (2R,6S,2"S)-10-Formylamino-3-[2(2,6-difluorphenyl-methoxy)-propyl] 1,2,3,4,5,6-hexahydro-6,11,11-trimethyl-2,6-methano-3-benzazocin-hydrochlorid:

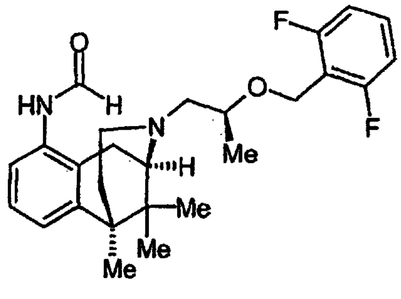

0,5 g (1,2 mmol) (2R,6S,2"S)-10-Amino-3-[2(2,6-difluorphenyl-methoxy)-propyl]-1,2,3,4,5,6-hexahydro-6,11,11-trimethyl-2,6-methano-3-benzazocin (Beispiel 1) werden mit 10 mL 97%iger Ameisensäure versetzt und 1 h unter Rückfluß gekocht. Anschließend wird i. Vak. eingeengt, der Rückstand mit Eis und 50 mL Ammoniak versetzt und je zweimal mit 50 mL Essigester extrahiert. Die vereinigte organische Phase wird mit 30 mL Wasser gewaschen, getrocknet und das Lösungsmittel i. Vak. abgezogen. Der Rückstand wird in Ether aufgenommen und mit etherischer HCl das Hydrochlorid gefällt. Ausbeute: 0,3 g (52%), Schmp.: amorph, $[\alpha]_D^{20}$ = (-) 41,6° (c=1 in MeOH).

**Beispiel 21:** (2R,6S,2"S)-10-Methylamino-3-[2(2,6-difluorphenyl-methoxy)-propyl]-1,2,3,4,5,6-hexahydro-6,11,11-trimethyl-2,6-methano-3-benzazocin-dihydrochlorid:

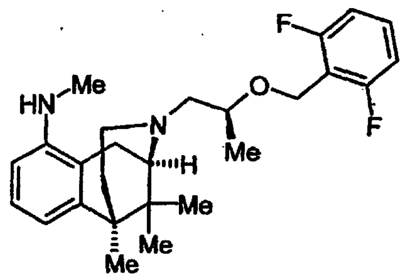

1,0 g (2,4 mmol) (2R,6S,2"S)-10-Amino-3-[2(2,6-difluorphenyl-methoxy)-propyl]-1,2,3,4,5,6-hexahydro-6,11,11-trimethyl-2,6-methano-3-benzazocin (Beispiel 1) werden in 10 mL Essigester gelöst und mit 1 mL Triethylamin und 1 mL Trifluoressigsäureanhydrid versetzt. Nach 10 min wird zweimal mit je 10 mL Wasser gewaschen, die organische Phase getrocknet und das Lösungsmittel i. Vak. abgezogen. Der Rückstand wird in 10 mL Dimethylacetamid aufgenommen und mit 0,5 g NaH versetzt. Nach 15 min. werden 1 mL Methyliodid zugegeben und 1 h bei 50 °C gerührt. Anschließend wird das Lösungsmittel i. Vak abgezogen, der Rückstand mit 20 mL Methanol und 2 mL 20%ige NaOH versetzt und 30 min. bei 60°C gerührt. Die Lösung wird erneut i. Vak. eingeengt, der Rückstand mit 50 mL Wasser versetzt und zweimal mit je 100 mL Ether extrahiert Die vereinigte organische Phase wird getrocknet, das Lösungsmittel i. Vak. abgezogen und der Rückstand an Kieselgel chromatographiert. Die geeigneten Fraktionen werden vereinigt und mit etherischer HCl das Hydrochlorid gefällt. Ausbeute: 0,4 g (35%), Schmp.: 243 °C, $[\alpha]_D^{20}$ = (-) 20,5° (c=1 in MeOH).

**Beispiel 22:** (2R,6S,2"S)-10-Dimethylamino-3-[2(2,6-difluorphenyl-methoxy)-propyl]-1,2,3,4,5,6-hexahydro-6,11,11-trimethyl-2,6-methano-3-benzazocin-dihydrochlorid:

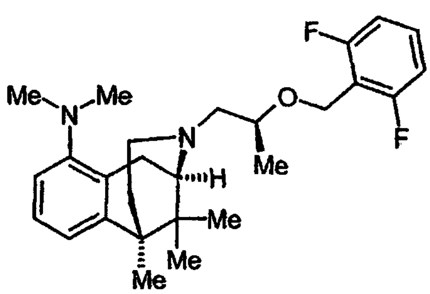

Zu einer auf -10 °C gekühlten Lösung aus 0,6 mL 37%iges Formalin und 1,2 mL 3N $H_2SO_4$ wird portionsweise eine Suspension aus 0,4 g (1 mmol) (2R,6S,2"S)-10-Amino-3-[2(2,6-difluorphenyl-methoxy)-propyl]-1,2,3,4,5,6-hexahydro-6,11,11-trimethyl-2,6-methano-3-benzazocin (Beispiel 1) und 0,3 g $NaBH_4$ in 7 mL THF gegeben. Nach beendeter Zugabe läßt man 10 min nachreagieren, versetzt mit Ammoniak und extrahiert zweimal mit je 20 mL Essigester. Die organische Phase wird getrocknet, das Lösungsmittel i. Vak. abgezogen, Der Rückstand in Ether aufgenommen und mit etherischer HCl das Hydrochlorid gefällt. Ausbeute: 0,5 g (97%), Schmp.: 125 °C, $[\alpha]_D^{20}$ = (-) 20,0° (c=1 in MeOH).

**Beispiel 23:** (2R,6S,2"S)-10-Ethylamino-3-[2(2,6-difluorphenyl-methoxy)-propyl]-1,2,3,4,5,6-hexahydro-6,11,11-trimethyl-2,6-methano-3-benzazocin-dihydrochlorid:

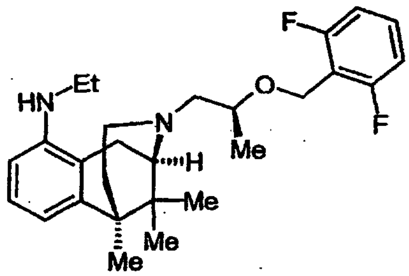

1,1 g (2,4 mmol) (2R,6S,2"S)-10-Acetamino-3-[2(2,6-difluorphenyl-methoxy)-propyl]-1,2,3,4,5,6-hexahydro-6,11,11-trimethyl-2,6-methano-3-benzazocin (Beispiel **1**) werden in 30 mL THF gelöst, mit 0,5 g $NaBH_4$ und 1,5 mL $BF_3$-Etherat versetzt und 6 h unter Rückfluß erhitzt. Man läßt abkühlen, fügt 30 mL MeOH und 60 mL 2 N HCl hinzu und erhitzt weitere 30 min unter Rückfluß. Anschließend wird das Lösungsmittel i. Vak. abgezogen, der Rückstand mit konz Ammoniak versetzt und zweimal mit je 30 mL Essigester extrahiert. Die vereinigte organische Phase wird einmal mit 20 mL Wasser gewaschen, getrocknet und das Lösungsmittel i. Vak. abgezogen. Der Rückstand wird in Aceton aufgenommen und mit etherischer HCl das Hydrochlorid gefällt. Ausbeute: 0,95 g (89%), Schmp.: 242 °C, $[\alpha]_D^{20}$ = (-) 24,8° (c=1 in MeOH).

**Beispiel 24:** (2R,6S,2"S)-10-Fluor-3-[2(2,6-difluorphenyl-methoxy)-propyl]-1,2,3,4,5,6-hexahydro-6,11,11-trimethyl-2,6-methano-3-benzazocin-hydrochlorid:

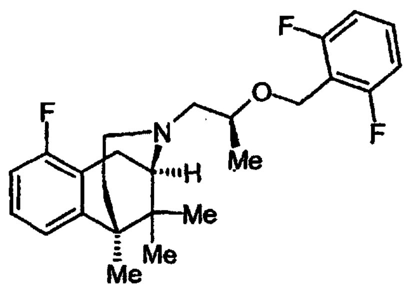

1,5 g (3,6 mmol) ) (2R,6S,2"S)-10-Amino-3-[2(2,6-difluorphenyl-methoxy)-propyl]-1,2,3,4,5,6-hexahydro-6,11,11-trimethyl-2,6-methano-3-benzazocin (Beispiel 1) werden in 30 mL Dioxan vorgelegt, mit 3 g $NOBF_4$ in 30 mL Dioxan versetzt und 1 h bei 90 °C gerührt. Anschließend wird das Lösungsmittel i. Vak. abgezogen, der Rückstand mit 50 mL Essigester versetzt und je einmal mit 30 mL verd. Ammoniak und 30 mL Wasser gewaschen. Die vereinigte organische Phase wird getrocknet, das Lösungsmittel i. Vak. abgezogen und der Rückstand an Kieselgel chromatographiert. Die geeigneten Fraktionen werden vereinigt und mit etherischer HCl das Hydrochlorid gefällt. Ausbeute: 0,5 g (33%), Schmp.: 190 °C, $[\alpha]_D^{20}$ = (-) 27,3° (c=1 in MeOH).

**Beispiel 25:** (2R,6S,2"S)-10-Amino-7-methyl-3-[2-(2,6-difluorphenyl-methoxy)-propyl]-1,2,3,4,5,6-hexahydro-6,11,11-trimethyl-2,6-methano-3-benzazocindihydrochlorid:

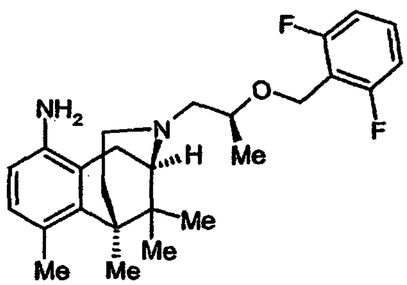

Herstellung ausgehend von **2s** in Analogie zu Beispiel 1; Schmp.: 236 °C;.

**Beispiel 26:** (2R,6S,2"S)-10-Amino-7-methyl-3-[2-(benzyloxy)propyl]-1,2,3,4,5,6-hexahydro-6,11,11-trimethyl-2,6-methano-3-benzazocin-dihydrochlorid:

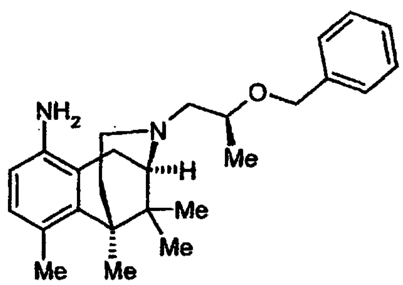

Herstellung ausgehend von **2t** in Analogie zu Beispiel 1; Schmp.: 227 °C, $[\alpha]_D^{20}$ = (-) 31,8° (c=1 in MeOH).

**Beispiel 27:** (2R,6S,2"S)-10-Formylamino-7-methyl-3-[2-(benzyloxy)propyl]-1,2,3,4,5,6-hexahydro-6,11,11-trimethyl-2,6-methano-3-benzazocin-dihydrochlorid:

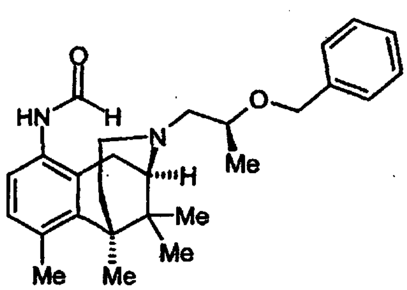

Herstellung ausgehend von Beispiel 26 in Analogie zu Beispiel 20; Schmp.: 141 °C;.

**Beispiel 28:** (2R,6S,2"S)-10-Formylamino-7-methyl-3-[2-(2,6-difluorphenylmethoxy)propyl]-1,2,3,4,5,6-hexahydro-6,11,11-trimethyl-2,6-methano-3-benzazocindihydrochlorid:

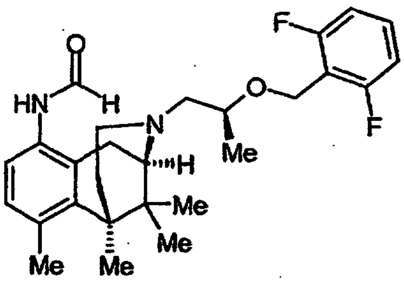

Herstellung ausgehend von Beispiel 28 in Analogie zu Beispiel 20; Schmp.: >105°C °C (Zers.), $[\alpha]_D^{20}$ = (-)42,2° (c=1 in MeOH);.

**Beispiel 29:** (2R,6S,2"S)-10-Methylamino-7-methyl-3-[2-(benzyloxy)propyl]-1,2,3,4,5,6-hexahydro-6,11,11-trimethyl-2,6-methano-3-benzazocin-dihydrochlorid:

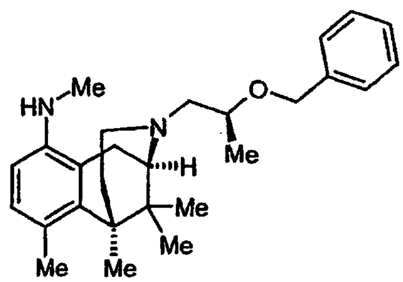

Herstellung ausgehend von Beispiel 26 in Analogie zu Beispiel 21; Schmp.: >100 °C (Zers.);.

**Beispiel 30:** (2R,6S,2"S)-10-Methylamino-7-methyl-3-[2-(2,6-difluorphenylmethoxy)propyl]-1,2,3,4,5,6-hexahydro-6,11,11-trimethyl-2,6-methano-3-benzazocindihydrobromid:

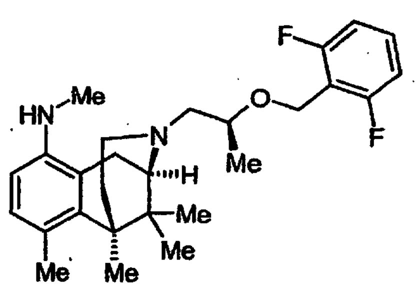

Herstellung ausgehend von Beispiel 25 in Analogie zu Beispiel 21; Schmp.: 221 °C (Zers.), $[\alpha]_D^{20}$ = (-)29,4° (c=1 in MeOH);.

**Beispiel 31:** (2R,6S,2"S)-10-Amino-8-methyl-3-[2-(benzyloxy)propyl]-1,2,3,4,5,6-hexahydro-6,11,11-trimethyl-2,6-methano-3-benzazocin-dihydrochlorid:

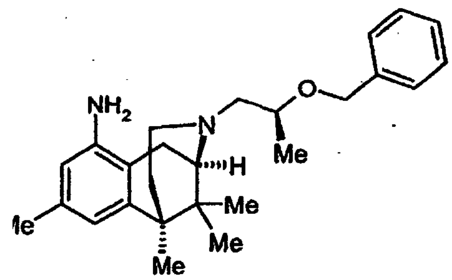

Herstellung anlog zu Beispiel 1; Schmp.: 250 °C;.

**Beispiel 32:** (2R,6S,2"S)-10-Amino-7-chlor-3-[2(benzyloxy)-propyl]-1,2,3,4,5,6-hexahydro-6,11,11-trimethyl-2,6-methano-3-benzazocin-dihydrochlorid:

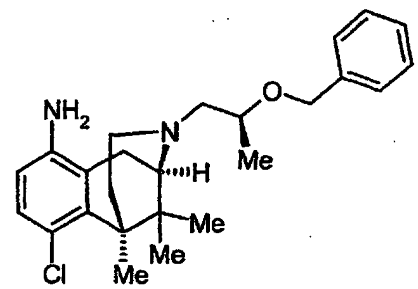

4,5 g (10 mmol) (2R,6S,2"S)-10-Amino-3-[2-(benzyloxy)propyl]-1,2,3,4,5,6-hexahydro-6,11,11-trimethyl-2,6-methano-3-benzazocin-dihydrochlorid (Beispiel 2) werden in 90 mL MeOH und 90 mL Eisessig gelöst, mit 1,45 g NCS (N-Chlorsuccinimid) versetzt und 4 h bei RT gerührt. Anschließend wird das Lösungsmittel i. Vak. abgezogen, der Rückstand in 100 mL wässeriger Ammoniaklösung aufgenommen und zweimal mit je 50 mL Essigester extrahiert. Die vereinigte organische Phase wird getrocknet, das Lösungsmittel i. Vak. abgezogen und der Rückstand an Kieselgel chromatographiert. Die geeigneten Fraktionen werden i. Vak. eingeengt und mit etherischer HCl das Hydrochlorid gefällt.
Ausbeute: 1,3 g (27%); Schmp.: 222 °C, $[\alpha]_D^{20}$ = (-)17,4° (c=1 in MeOH);.

**Beispiel 33:** (2R,6S,2"S)-10-Amino-7,9-dichlor-3-[2(benzyloxy)-propyl]-1,2,3,4,5,6-hexahydro-6,11,11-trimethyl-2,6-methano-3-benzazocin-dihydrochlorid:

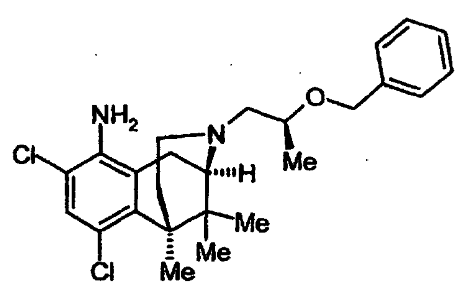

Herstellung gemeinsam mit Beispiel 32;
Nach chromatographischer Trennung werden erhalten:
Ausbeute: 0,6 g(12%); Schmp.: 197 °C, $[\alpha]_D^{20}$ = 11,0° (c=1 in MeOH);.

**Beispiel 34:** (2R,6S,2"S)-10-Amino-7-chlor-3-[2(2,6-difluorphenyl-methoxy)-propyl]-1,2,3,4,5,6-hexahydro-6,11,11-trimethyl-2,6-methano-3-benzazocin-dihydrochlorid:

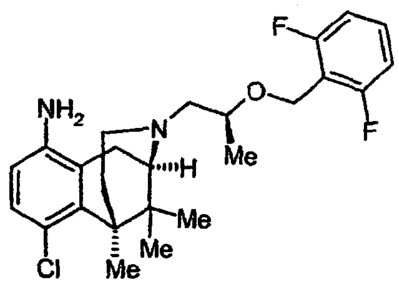

Herstellung ausgehend von Beispiel 1 in Analogie zu Beispiel 32;
Schmp.: 247 °C;.

**Beispiel 35:** (2R,6S,2"S)-10-Amino-7-brom-3-[2(benzyloxy)-propyl]-1,2,3,4,5,6-hexahydro-6,11,11-trimethyl-2,6-methano-3-benzazocin-dihydrochlorid:

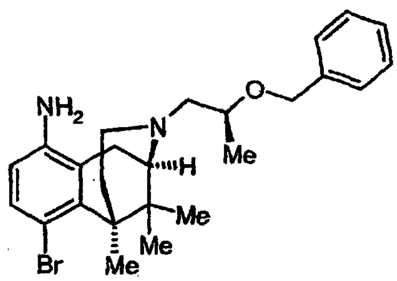

Herstellung ausgehend von Beispiel 2 in Analogie zu Beispiel 32 unter Verwendung von NBS;
Schmp.: 215 °C, $[\alpha]_D^{20}$ = (-)17,3° (c=1 in MeOH);.

**Beispiel 36:** (2R,6S,2"S)-10-Amino-7,9-dibrom-3-[2(benzyloxy)-propyl]-1,2,3,4,5,6-hexahydro-6,11,11-trimethyl-2,6-methano-3-benzazocin-dihydrochlorid:

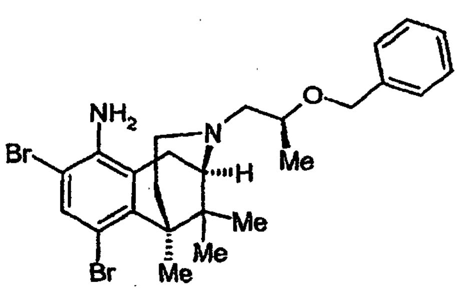

Herstellung gemeinsam mit Beispiel 35 in Analogie zu Beispiel 33;

Schmp.: 177 °C, $[\alpha]_D^{20}$ = 11,5° (c=1 in MeOH);.

**Beispiel 37:** (2R,6S,2"S)-10-Amino-7-brom-3-[2(2,6-difluorphenyl-methoxy)-propyl]-1,2,3,4,5,6-hexahydro-6,11,11-trimethyl-2,6-methano-3-benzazocin-dihydrochlorid:

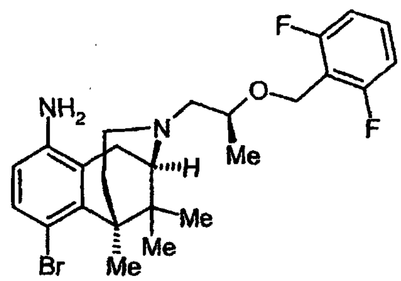

Herstellung ausgehend von Beispiel 1 in Analogie zu Beispiel 32 unter Verwendung von NBS; Schmp.: 233 °C, $[\alpha]_D^{20}$ = (-)20,5° (c=1 in MeOH);.

**Beispiel 38:** (2R,6S,2"S)-10-Amino-7,9-dibrom-3-[2(2,6-difluorphenyl-methoxy)-propyl]-1,2,3,4,5,6-hexahydro-6,11,11-trimethyl-2,6-methano-3-benzazocindihydrochlorid:

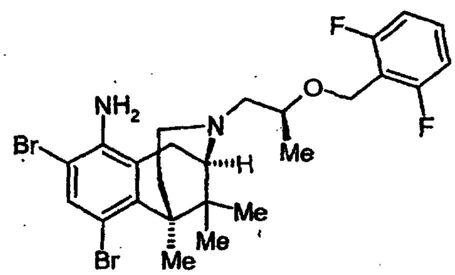

Herstellung gemeinsam mit Beispiel 37 in Analogie zu Beispiel 36; Schmp.: 236 °C, $[\alpha]_D^{20}$ = 5,9° (c=1 in MeOH);.

**Beispiel 39:** (2R,6S,2"S)-10-Amino-9-brom-3-[2(2,6-difluorphenyl-methoxy)-propyl]-1,2,3,4,5,6-hexahydro-6,11,11-trimethyl-2,6-methano-3-benzazocin-dihydrochlorid:

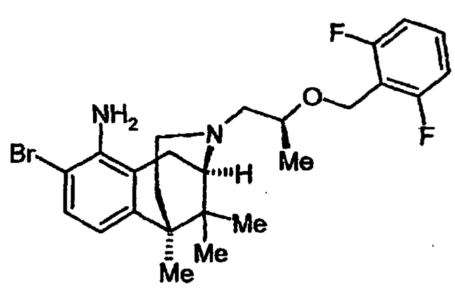

Herstellung gemeinsam mit Beispiel 37 in Analogie zu Beispiel 32; Schmp.: 252 °C, $[\alpha]_D^{20}$ = (-)14,6° (c=1 in MeOH);.

**Beispiel 40:** (2R,6S,2"S)-10-Amino-7-fluor-3-[2-(benzyloxy)propyl]-1,2,3,4,5,6-hexahydro-6,11,11-trimethyl-2,6-methano-3-benzazocin-dihydrochlorid:

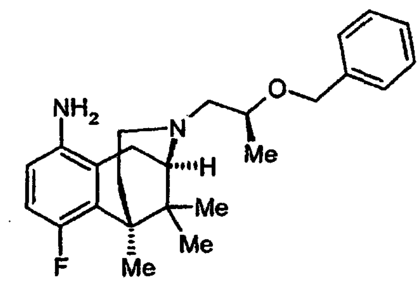

Herstellung ausgehend von **2v** in Analogie zu Beispiel 1;
Schmp.: 150 °C;.

**Beispiel 41:** (2R,6S,2"S,5"S)-10-Amino-7-methyl-3-[5"-phenyl-tetrahydrofuran-2"yl)methyl]-1,2,3,4,5,6-hexahydro-6,11,11-trimethyl-2,6-methano-3-benzazocindihydrochlorid:

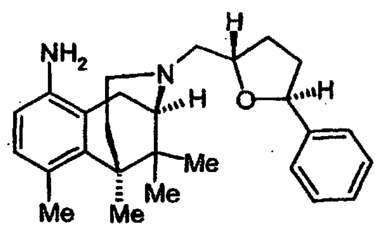

Herstellung ausgehend von **2u** in Analogie zu Beispiel 1;
Schmp.: 250 °C;.

**Beispiel 42:** (2R,6S,2"S,5"S)-10-Methylamino-7-methyl-3-[5"-phenyl-tetrahydrofuran-2"-yl)methyl]-1,2,3,4,5,6-hexahydro-6,11,11-trimethyl-2,6-methano-3-benzazocindihydrochlorid:

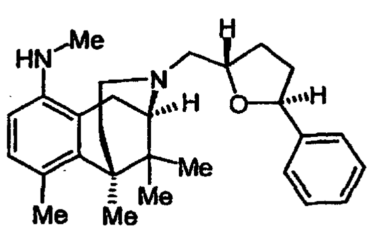

Herstellung ausgehend von Beispiel 41 in Analogie zu Beispiel 21;
Schmp.: 250 °C;.

**Beispiel 43:** (2R,6S,2"S,5"S)-10-Formylamino-7-methyl-3-[5"-phenyltetrahydrofuran-2"-yl)methyl]-1,2,3,4,5,6-hexahydro-6,11,11-trimethyl-2,6-methano-3-benzazocin-dihydrochlorid:

Herstellung ausgehend von Beispiel 41 in Analogie zu Beispiel 20;
Schmp.: 250 °C;.

**Beispiel 44:** (2R,6S,2"S)-10-Amino-7-trifluormethyl-3-[2(benzyloxy)-propyl]-1,2,3,4,5,6-hexahydro-6-methyl-2,6-methano-3-benzazocin-dihydrochlorid:

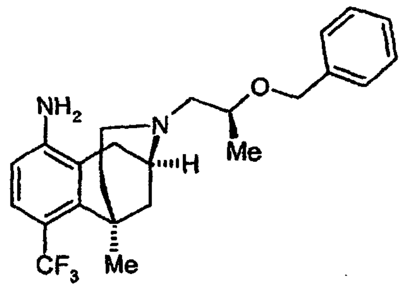

Herstellung ausgehend von **2w** in Analogie zu Beispiel 1;
Schmp.: 165 °C;.

**Beispiel 45:** (2R,6S,2"S)-10-Amino-7-(4-methylphenyl)-3-[2(2,6-difluorphenylmethoxy)-propyl]-1,2,3,4,5,6-hexahydro-6-methyl-2,6-methano-3-benzazocindihydrochlorid:

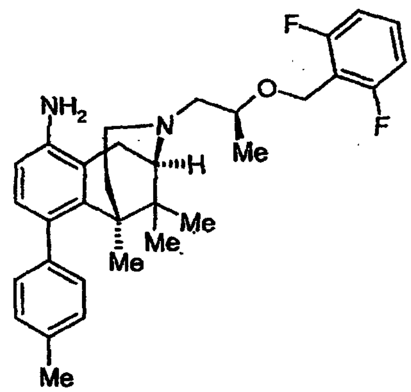

Herstellung ausgehend von **2x** in Analogie zu Beispiel 1;
Schmp.: 219 °C, $[\alpha]_D^{20}$ = (-)19,5° (c=1 in MeOH).

Die erfindungsgemäßen Verbindungen können oral, transdermal, nasal, inhalativ oder parenteral verabreicht werden. Die erfindungsgemäßen Verbindungen liegen hierbei als aktive Bestandteile in üblichen Darreichungsformen vor, beispielsweise in Zusammensetzungen, die im wesentlichen aus einem inerten pharmazeutischen Träger und einer effektiven Dosis des Wirkstoffs bestehen, wie beispielsweise Tabletten, Dragées, Kapseln, Oblaten, Pulver, Lösungen, Suspensionen, Emulsionen, Sirupe, Suppositorien, transdermale Systeme etc.. Eine wirksame Dosis der erfindungsgemäßen Verbindungen liegt bei einer oralen Anwendung zwischen 1 und 1000, vorzugsweise zwischen 1 und 500, besonders bevorzugt zwischen 5-300 mg/Dosis, bei intravenöser, subcutaner oder intramuskulärer Anwendung zwischen 0,001 und 50, vorzugsweise zwischen 0,1 und 10 mg/Dosis. Für die Inhalation sind erfindungsgemäß Lösungen geeignet, die 0,01 bis 1,0, vorzugsweise 0,1 bis 0,5 % Wirkstoff enthalten. Für die inhalative Applikation ist die Verwendung von Pulvern bevorzugt. Gleichfalls ist es möglich, die erfindungsgemäßen Verbindungen als Infusionslösung, vorzugsweise in einer physiologischen Kochsalzlösung oder Nährsalzlösung einzusetzen.

Die erfindungsgemäßen Verbindungen können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbessemdes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägem, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Eine therapeutisch wirksame Tagesdosis beträgt zwischen 1 und 1000 mg, bevorzugt 10 - 500 mg pro Erwachsener.

Die nachfolgenden Beispiele illustrieren die vorliegende Erfindung ohne sie jedoch in ihrem Umfang zu beschränken:

Pharmazeutische Formulierungsbeispiele

A)

| Tabletten | pro Tablette |
|---|---|
| Wirkstoff | 300 mg |
| Milchzucker | 240 mg |
| Maisstärke | 340 mg |
| Polyvinylpyrrolidon | 45 mg |
| Magnesiumstearat | 15 mg |
| | 940 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

B)

| Tabletten | pro Tablette |
|---|---|
| Wirkstoff | 80 mg |
| Maisstärke | 190 mg |
| Milchzucker | 55 mg |
| Mikrokristalline Cellulose | 35 mg |
| Polyvinylpyrrolidon | 15 mg |
| Natrium-carboxymethylstärke | 23 mg |
| Magnesiumstearat | 2 mg |

(fortgesetzt)

| Tabletten | pro Tablette |
|---|---|
| | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium-carboxymethylstärke und das Magnesiumstearat, vermischt und verpreßt das Gemisch zu Tabletten geeigneter Größe.

C)

| Dragées | pro Dragée |
|---|---|
| Wirkstoff | 5 mg |
| Maisstärke | 41,5 mg |
| Milchzucker | 30 mg |
| Polyvinylpyrrolidon | 3 mg |
| Magnesiumstearat | 0,5 mg |
| | 80 mg |

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichten aus Zucker und Talkum besteht. Die fertigen Dragées werden mit Wachs poliert.

D)

| Kapseln | pro Kapsel |
|---|---|
| Wirkstoff | 150 mg |
| Maisstärke | 268,5 mg |
| Magnesiumstearat | 1,5 mg |
| | 420 mg |

Substanz und Maisstärke werden gemischt und mit Wasser befeuchtet. Die feuchte Masse wird gesiebt und getrocknet. Das trockene Granulat wird gesiebt und mit Magensiumstearat gemischt. Die Endmischung wird in Hartgelatinekapseln Größe 1 abgefüllt.

E)

| E) Ampullenlösung | |
|---|---|
| Wirkstoff | 50 mg |
| Natriumchlorid | 50 mg |
| Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt. die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

F)

| Suppositorien | |
|---|---|
| Wirkstoff | 50 mg |
| Adeps solidus | 1650 mg |
| | 1700 mg |

Das Hartfett wird geschmolzen. Bei 40°C wird die gemahlene Wirksubstanz homogen dispergiert Es wird auf 38°C abgekühlt und in schwach vorgekühlte Suppositorienformen ausgegossen.

## Patentansprüche

**1.** Verbindungen der allgemeinen Formel 1

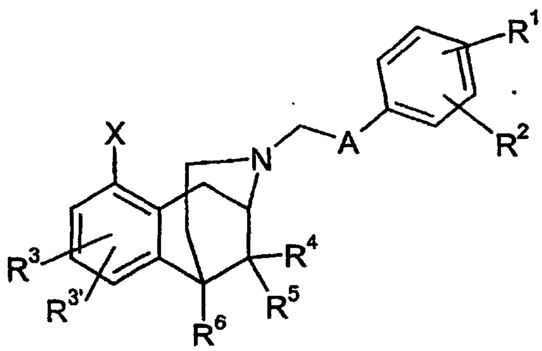

1

worin

| | |
|---|---|
| $R^1$ und $R^2$ | gleich oder verschieden Wasserstoff, $C_1$-$C_6$-Alkyl, $C_1$-$C_6$-Alkyloxy, OH, F, Cl oder Br; |
| $R^3$ und $R^{3'}$ | gleich oder verschieden Wasserstoff, F, Cl, Br, Methyl, Ethyl, OH, $CF_3$, Methoxy oder Phenyl, welches gegebenenfalls substituiert sein kann durch einen Rest ausgewählt aus der Gruppe F, Cl, Br, Methyl, Ethyl, OH, $CF_3$ und Methoxy; |
| $R^4$, $R^5$ und $R^6$ | gleich oder verschieden Wasserstoff, Methyl oder Ethyl, |
| X | $NH_2$, NH-( $C_1$-$C_6$-Alkyl), N($C_1$-$C_6$-Alkyl)$_2$, wobei die beiden $C_1$-$C_6$-Alkyl-Gruppen gleich oder verschieden sein können, NH-COH, NH-CO($C_1$-$C_6$-Alkyl) oder F; |
| A | $-(CH_2)_3-$, $-CH_2-CH_2-O-$, $-CH_2-O-CH_2-$, $-(CH_2)_4-$, -CH($C_1$-$C_6$-Alkyl)-O-$CH_2$-, $-(CH_2)_2-O-CH_2-$, $-(CH_2)_3-O-$, $-(CH_2)_5-$, $-CH_2-O-(CH_2)_3-$, $-(CH_2)_2-O-(CH_2)_2-$, $-(CH_2)_3-O-CH_2-$, $-(CH_2)_4-O-$, $-CH_2-O-CH_2-CH_2-O-$, |

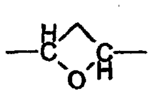 , 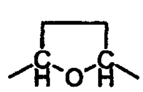 oder 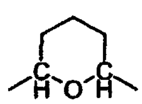

bedeuten können, gegebenenfalls in Form ihrer Racemate, ihrer Enantiomere, ihrer Diastereomere und ihrer Gemische, sowie gegebenenfalls ihrer pharmakologisch unbedenklichen Säureadditionssalze.

**2.** Verbindungen der allgemeinen Formel 1 nach Anspruch 1, worin

| | |
|---|---|
| $R^1$ und $R^2$ | gleich oder verschieden Wasserstoff, Methyl, Ethyl, Methyloxy, Ethyloxy, OH, F, Cl oder Br; |

$R^3$ und $R^{3'}$ gleich oder verschieden Wasserstoff, F, Cl, Br, Methyl, Ethyl, OH, $CF_3$, Methoxy oder Phenyl, welches durch einen Rest ausgewählt aus der Gruppe F, Cl, Br und bevorzugt Methyl, substituiert ist;

$R^4$, $R^5$ und $R^6$ gleich oder verschieden Wasserstoff oder Methyl;

X $NH_2$, NH-(Methyl), $N(Methyl)_2$, NH-(Ethyl), $N(Ethyl)_2$, NH-COH, NH-COMe oder F;

A $-CH_2-CH_2-O-$, $-CH_2-O-CH_2-$, $-CH(Methyl)-O-CH_2-$, $-CH(Ethyl)-O-CH_2-$, $-CH(iso\text{-}Propyl)-O-CH_2-$, $-(CH_2)_2-O-CH_2-$, $-(CH_2)_3-O-$, $-CH_2-O-(CH_2)_3-$, $-(CH_2)_2-O-(CH_2)_2-$, $-(CH_2)_3-O-CH_2-$, $-(CH_2)_4-O-$, $-CH_2-O-CH_2-CH_2-O-$,

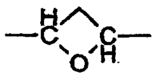 , 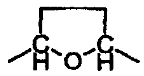 oder 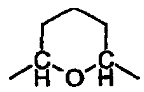

bedeuten können, gegebenenfalls in Form ihrer Racemate, ihrer Enantiomere, ihrer Diastereomere und ihrer Gemische, sowie gegebenenfalls ihrer pharmakologisch unbedenklichen Säureadditionssalze.

**3.** Verbindungen der allgemeinen Formel <u>**1**</u> nach Anspruch 1 oder 2, worin

$R^1$und $R^2$ gleich oder verschieden Wasserstoff oder F;
$R^3$ und $R^{3'}$ gleich oder verschieden Wasserstoff, F, Cl, Br, $CF_3$ oder Methyl;
$R^4$, $R^5$ und $R^6$ gleich oder verschieden Wasserstoff oder Methyl;
X $NH_2$, NH-(Methyl), $N(Methyl)_2$, NH-COH, NH-COMe oder F;
A $-CH(Methyl)-O-CH_2-$, $-CH_2-O-CH_2-$ oder

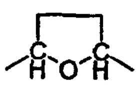

bedeuten können, gegebenenfalls in Form ihrer Racemate, ihrer Enantiomere, ihrer Diastereomere und ihrer Gemische, sowie gegebenenfalls ihrer pharmakologisch unbedenklichen Säureadditionssalze.

**4.** Verbindungen der allgemeinen Formel **1** nach Anspruch 1, 2 oder 3, worin

$R^1$ und $R^2$ gleich oder verschieden Wasserstoff oder F;
$R^3$ und $R^{3'}$ gleich oder verschieden Wasserstoff, F, Cl, Br, $CF_3$ oder Methyl;
$R^4$, $R^5$ und $R^6$ gleich oder verschieden Wasserstoff oder Methyl;
X $NH_2$, NH-(Methyl) oder NH-COH;
A $-CH(Methyl)-O-CH_2-$, $-CH_2-O-CH_2-$ oder

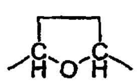

bedeuten können, gegebenenfalls in Form ihrer Racemate, ihrer Enantiomere, ihrer Diastereomere und ihrer Gemische, sowie gegebenenfalls ihrer pharmakologisch unbedenklichen Säureadditionssalze.

**5.** Verbindungen der allgemeinen Formel **1** nach Anspruch 1, 2 oder 3, worin

$R^1$ und $R^2$ gleich oder verschieden Wasserstoff oder F;
$R^3$ und $R^{3'}$ Wasserstoff;

| | |
|---|---|
| $R^4$, $R^5$ und $R^6$ | gleich oder verschieden Wasserstoff oder Methyl; |
| X | F; |
| A | -CH(Methyl)-O-$CH_2$-, |

bedeuten können, gegebenenfalls in Form ihrer Racemate, ihrer Enantiomere, ihrer Diastereomere und ihrer Gemische, sowie gegebenenfalls ihrer pharmakologisch unbedenklichen Säureadditionssalze.

6. Pharmazeutische Zubereitung **gekennzeichnet durch** einen Gehalt an einer der Verbindungen der Formel 1 nach einem der Ansprüche 1 bis 5 neben üblichen Hilfs- und Trägerstoffen.

7. Verwendung einer der Verbindungen der Formel **1** nach einem der Ansprüche 1 bis 5 als Arzneimittel.

8. Verwendung einer der Verbindungen der Formel **1** nach einem der Ansprüche 1 bis 5 zur Herstellung eines Arzneimittels zur Vorbeugung oder Behandlung von Erkrankungen oder Störungen, bei denen die Blockade des spannungsabhängigen Natriumkanals einen therapeutischen Nutzen entfalten kann.

9. Verwendung einer der Verbindungen der Formel **1** nach einem der Ansprüche 1 bis 5 zur Herstellung eines Arzneimittels zur Vorbeugung oder Behandlung von Arrhythmien, Spasmen, kardialen und Gehimischämien, Schmerzen sowie neurodegenerative Erkrankungen.

10. Verwendung einer der Verbindungen der Formel **1** nach einem der Ansprüche 1 bis 5 zur Herstellung eines Arzneimittels zur Vorbeugung oder Behandlung von Epilepsie, Hypoglykämie, Hypoxie, Anoxie, Gehimtrauma, Gehimoedem, Gehim-Schlaganfall, perinatale Asphyxie, Degenerationen des Cerebellums, amyotrope laterale Sklerose, Morbus Huntington, Morbus Alzheimer, Morbus Parkinson, Zyklophrenie, Hypotonie, Herzinfakt, Herzrhythmusstörungen, Angina pectoris, chronischer Schmerz, neuropathischer Schmerz oder Lokalanaesthesie.

11. Verfahren zur Herstellung von Verbindungen der allgemeinen Formel **1**

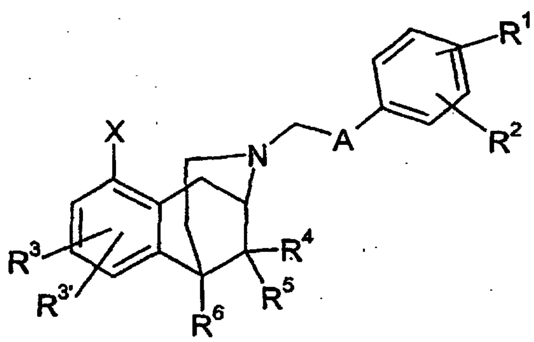

**1**

worin die Reste $R^1$, $R^2$, $R^3$, $R^{3'}$, $R^4$, $R^5$, $R^6$ und A die in den Ansprüchen 1-5 genannten Bedeutungen haben können und X für $NH_2$ steht, **dadurch gekennzeichnet, daß** Verbindungen der allgemeinen Formel **3**

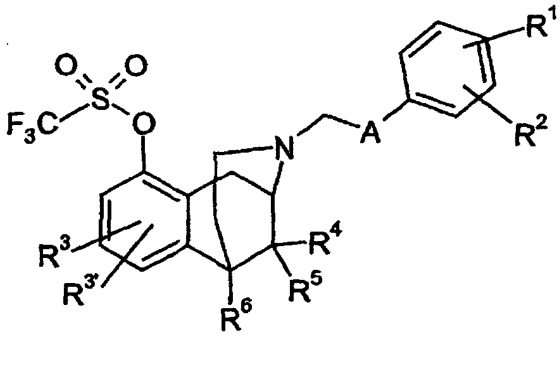

**3**

worin die Reste $R^1$, $R^2$, $R^3$, $R^{3'}$, $R^4$, $R^5$, $R^6$ und A die in den Ansprüchen 1-5 genannten Bedeutungen haben

können, in einem aromatischen Lösungsmittel unter Palladium-Katalyse mit einer Stickstoffquelle umgesetzt werden.

**12.** Zwischenverbindungen der allgemeinen Formel 3

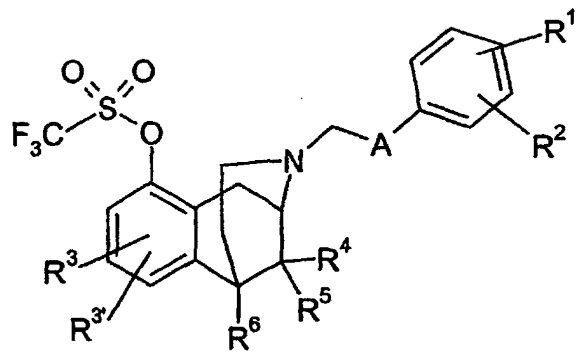

3

worin die Reste $R^1$, $R^2$, $R^3$, $R^{3'}$, $R^4$, $R^5$, $R^6$ und A die in den Ansprüchen 1-5 genannten Bedeutungen haben können.

**13.** Verfahren zur Herstellung von Verbindungen der allgemeinen Formel 1

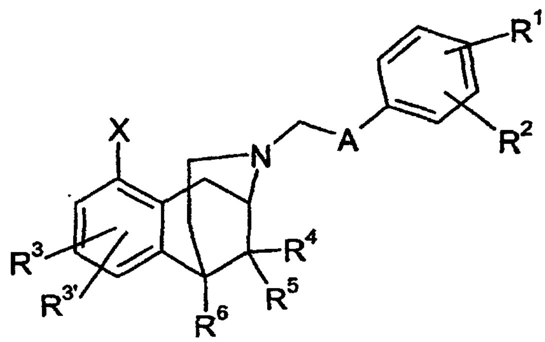

1

worin die Reste $R^1$, $R^2$, $R^3$, $R^{3'}$, $R^4$, $R^5$, $R^6$ und A die in den Ansprüchen 1-5 genannten Bedeutungen haben können, **dadurch gekennzeichnet, daß** Verbindungen der allgemeinen Formel 1 in denen X für $NH_2$ steht

a) in einem polaren organischen Lösemittel, mit einer Base und einem entsprechenden Alkylierungsmittel zu Verbindungen der allgemeinen Formel 1, in denen X für NH-( $C_1$-$C_6$-Alkyl) oder N($C_1$-$C_6$-Alkyl)$_2$ steht, umgesetzt werden, oder
b) unter Kühlung, in Gegenwart von Säuren mit Aldehyden oder Ketonen umgesetzt werden und die so intermediär gebildeten Schiffschen Basen oder Iminiumsalze anschließend mit Metallhydriden zu Verbindungen der allgemeinen Formel 1, in denen X für NH-($C_1$-$C_6$-Alkyl) oder N($C_1$-$C_6$-Alkyl)$_2$ steht reduziert werden, oder
c) in Gegenwart einer Base mit einem Säurechlorid oder Anhydrid zu Verbindungen der allgemeinen Formel 1, in denen X für NHCO( $C_1$-$C_6$-Alkyl) steht umgesetzt werden, oder
d) gegebenenfalls die gemäß Stufe c) erhaltenen Verbindungen der Formel 1, in denen X für NHCO( $C_1$-$C_6$-Alkyl) steht, anschließend mit Metallhydriden unter Lewis-Säure-Katalyse zu Verbindungen der allgemeinen Formel 1, in denen X für NH-( $C_1$-$C_6$-Alkyl) oder N($C_1$-$C_6$-Alkyl)$_2$ steht, reduziert werden, oder
e) bei erhöhter Temperatur durch Umsetzung mit Ameisensäure in die Verbindungen der allgemeinen Formel 1, in denen X für NHCOH steht überführt werden, oder
f) durch Diazotierung von und anschließender Verkochung mit $BF_4^-$ zu den Verbindungen der Formel 1 in denen X für F steht, umgesetzt werden.

## Claims

**1.** Compounds of general formula 1

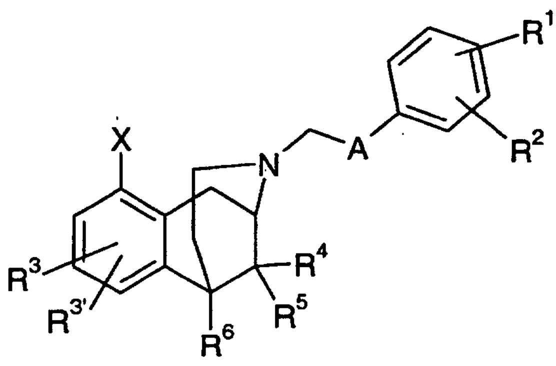

1

wherein

| | |
|---|---|
| $R^1$ and $R^2$ | which may be identical or different may denote hydrogen, $C_1$-$C_6$-alkyl, $C_1$-$C_6$-alkyloxy, OH, F, Cl or Br; |
| $R^3$ and $R^{3'}$ | which may be identical or different may denote hydrogen, F, Cl, Br, methyl, ethyl, OH, $CF_3$, methoxy or phenyl, which may optionally be substituted by a group selected from among F, Cl, Br, methyl, ethyl, OH, $CF_3$ and methoxy; |
| $R^4$, $R^5$ and $R^6$ | which may be identical or different may denote hydrogen, methyl or ethyl, |
| X | may denote $NH_2$, NH-($C_1$-$C_6$-alkyl), N($C_1$-$C_6$-alkyl)$_2$, the two $C_1$-$C_6$-alkyl groups of which may be identical or different, NH-COH, NH-CO($C_1$-$C_6$-alkyl) or F; |
| A | may denote $-(CH_2)_3-$, $-CH_2-CH_2-O-$, $-CH_2-O-CH_2-$, $-(CH_2)_4-$, $-CH(C_1-C_6\text{-alkyl})-O-CH_2-$, $-(CH_2)_2-O-CH_2-$, $-(CH_2)_3-O-$, $-(CH_2)_5-$, $-CH_2-O-(CH_2)_3-$, $-(CH_2)_2-O-(CH_2)_2-$, $-(CH_2)_3-O-CH_2-$, $-(CH_2)_4-O-$, $-CH_2-O-CH_2-CH_2-O-$, |

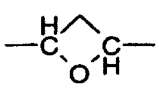 , 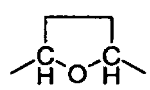 or 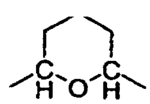 .

optionally in the form of their racemates, their enantiomers, their diastereomers and mixtures thereof, and optionally the pharmacologically acceptable acid addition salts thereof.

**2.** Compounds of general formula 1 according to claim 1,
wherein

| | |
|---|---|
| $R^1$ and $R^2$ | which may be identical or different denote hydrogen, methyl, ethyl, methyloxy, ethyloxy, OH, F, Cl or Br; |
| $R^3$ and $R^{3'}$ | which may be identical or different denote hydrogen, F, Cl, Br, methyl, ethyl, OH, $CF_3$, methoxy or phenyl, which may optionally be substituted by a group selected from among F, Cl, Br and preferably methyl; |
| $R^4$, $R^5$ and $R^6$ | which may be identical or different may denote hydrogen or methyl; |
| X | may denote $NH_2$, NH-(methyl), N(methyl)$_2$, NH-(ethyl), N(ethyl)$_2$, NH-COH, NH-COMe or F; |
| A | may denote $-CH_2-CH_2-O-$, $-CH_2-O-CH_2-$, -CH(methyl)-O-$CH_2$-, -CH(ethyl)-O-$CH_2$-, -CH(isopropyl)-O-$CH_2$-, $-(CH_2)_2-O-CH_2-$, $-(CH_2)_3-O-$, $-CH_2-O-(CH_2)_3-$, $-(CH_2)_2-O-(CH_2)_2-$, $-(CH_2)_3-O-CH_2-$, $-(CH_2)_4-O-$, $-CH_2-O-CH_2-CH_2-O-$, |

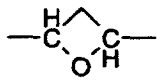 , 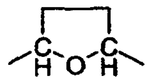 or 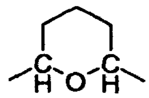

optionally in the form of their racemates, their enantiomers, their diastereomers and mixtures thereof, and optionally the pharmacologically acceptable acid addition salts thereof.

**3.** Compounds of general formula **1** according to claim 1 or 2,
wherein

| | |
|---|---|
| $R^1$ and $R^2$ | which may be identical or different may denote hydrogen or F; |
| $R^3$ and $R^{3'}$ | which may be identical or different may denote hydrogen, F, Cl, Br, $CF_3$ or methyl; |
| $R^4$, $R^5$ and $R^6$ | which may be identical or different may denote hydrogen or methyl; |
| X | may denote $NH_2$, NH-(methyl), N(methyl)$_2$, NH-COH, NH-COMe or F; |
| A | may denote -CH(methyl)-O-$CH_2$-, -$CH_2$-O-$CH_2$- or |

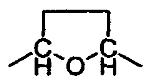

optionally in the form of their racemates, their enantiomers, their diastereomers and mixtures thereof, and optionally the pharmacologically acceptable acid addition salts thereof.

**4.** Compounds of general formula **1** according to claim 1, 2 or 3,
wherein

| | |
|---|---|
| $R^1$ and $R^2$ | which may be identical or different may denote hydrogen or F; |
| $R^3$ and $R^{3'}$ | which may be identical or different may denote hydrogen, F, Cl, Br, $CF_3$ or methyl; |
| $R^4$, $R^5$ and $R^6$ | which may be identical or different may denote hydrogen or methyl; |
| X | may denote $NH_2$, NH-(methyl) or NH-COH; |
| A | may denote -CH(methyl)-O-$CH_2$-, -$CH_2$-O-$CH_2$- or |

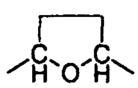

optionally in the form of their racemates, their enantiomers, their diastereomers and mixtures thereof, and optionally the pharmacologically acceptable acid addition salts thereof.

**5.** Compounds of general formula **1** according to claim 1, 2 or 3, wherein

| | |
|---|---|
| $R^1$ and $R^2$ | which may be identical or different may denote hydrogen or F; |
| $R^3$ and $R^{3'}$ | may denote hydrogen; |
| $R^4$, $R^5$ and $R^6$ | which may be identical or different may denote hydrogen or methyl; |

X may denote F;

A may denote $-CH(methyl)-O-CH_2-$,

optionally in the form of their racemates, their enantiomers, their diastereomers and mixtures thereof, and optionally the pharmacologically acceptable acid addition salts thereof.

6. Pharmaceutical preparation, **characterised in that** it contains one of the compounds of formula **1** according to one of claims 1 to 5 together with conventional excipients and carriers.

7. Use of one of the compounds of formula **1** according to one of claims 1 to 5 as a pharmaceutical composition.

8. Use of one of the compounds of formula **1** according to one of claims 1 to 5 for preparing a pharmaceutical composition for the prevention or treatment of diseases or disorders in which blockade of the voltage-dependent sodium channel may be of therapeutic value.

9. Use of one of the compounds of formula **1** according to one of claims 1 to 5 for preparing a pharmaceutical composition for the prevention or treatment of arrhythmias, spasms, cardiac and cerebral ischaemia, pain and neurodegenerative diseases.

10. Use of one of the compounds of formula **1** according to one of claims 1 to 5 for preparing a pharmaceutical composition for the prevention or treatment of epilepsy, hypoglycaemia, hypoxia, anoxia, brain trauma, brain oedema, cerebral stroke, perinatal asphyxia,' degeneration of the cerebellum, amyotropic lateral sclerosis, Huntington's disease, Alzheimer's disease, Parkinson's disease, cyclophrenia, hypotonia, cardiac infarct, cardiac rhythm disorders, angina pectoris, chronic pain, neuropathic pain or local anaesthesia.

11. Process for preparing compounds of general formula **1**

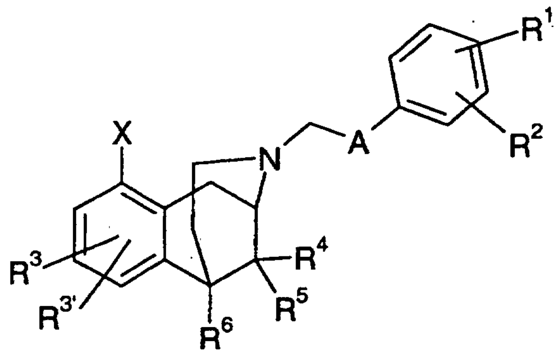

**1**

wherein the groups $R^1$, $R^2$, $R^3$, $R^{3'}$, $R^4$, $R^5$, $R^6$ and A have the meanings given in claims 1 to 5 and X denotes $NH_2$, **characterised in that** compounds of general formula **3**

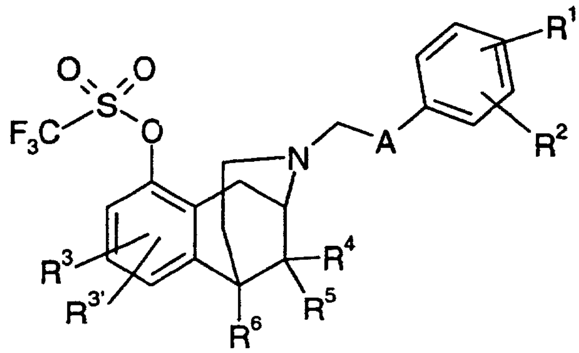

**3**

wherein the groups $R^1$, $R^2$, $R^3$, $R^{3'}$, $R^4$, $R^5$, $R^6$ and A may have the meanings given in claims 1-5, are reacted with a source of nitrogen in an aromatic solvent with palladium catalysis.

**12.** Intermediate compounds of general formula 3

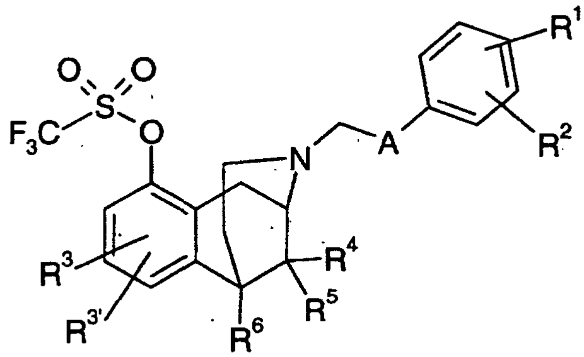

**3**

wherein the groups $R^1$, $R^2$, $R^3$, $R^{3'}$, $R^4$, $R^5$, $R^6$ and A may have the meanings given in claims 1 to 5.

**13.** Process for preparing compounds of general formula 1

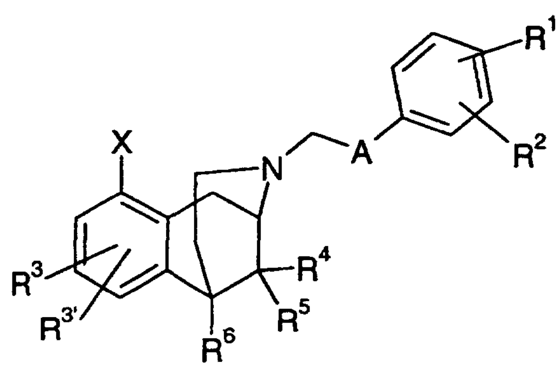

**1**

wherein the groups $R^1$, $R^2$, $R^3$, $R^{3'}$, $R^4$, $R^5$, $R^6$ and A may have the meanings given in claims 1-5, **characterised in that** compounds of general formula 1 wherein X denotes $NH_2$

a) are reacted with a base and a corresponding alkylating agent, in a polar organic solvent, to form compounds

of general formula **1** wherein X denotes NH-( $C_1$-$C_6$-alkyl) or N($C_1$-$C_6$-alkyl)$_2$, or

b) are reacted with aldehydes or ketones, with cooling, in the presence of acids, and the Schiff bases or iminium salts thus formed as intermediates are subsequently reduced with metal hydrides to form compounds of general formula **1** wherein X denotes NH-($C_1$-$C_6$-alkyl) or N($C_1$-$C_6$-alkyl)$_2$, or

c) are reacted, in the presence of a base, with an acid chloride or anhydride to form compounds of general formula **1** wherein X denotes NHCO($C_1$-$C_6$-alkyl), or

d) the compounds of formula **1** wherein X denotes NHCO($C_1$-$C_6$-alkyl), obtained according to step c), are then optionally reduced with metal hydrides, using Lewis acids as catalysts, to obtain compounds of general formula **1** wherein X denotes NH-( $C_1$-$C_6$-alkyl) or N($C_1$-$C_6$-alkyl)$_2$, or

e) are converted at elevated temperature into the compounds of general formula **1** wherein X denotes NHCOH by reacting with formic acid, or

f) are reacted by diazotisation and subsequent decoction with $BF_4^-$ to form the compounds of formula **1** wherein X denotes F.

**Revendications**

**1.** Composés de formule générale 1

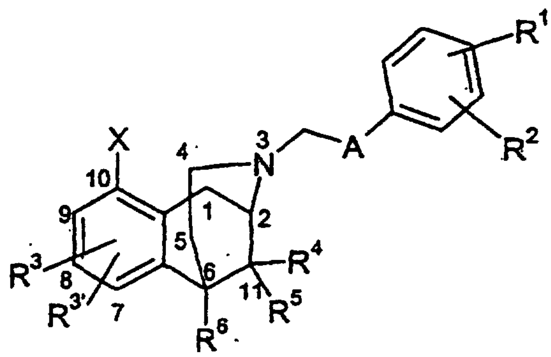

**1**

où

$R^1$ et $R^2$, identiques ou différents, peuvent représenter l'hydrogène, alkyle en $C_1$-$C_6$, alkyloxy en $C_1$-$C_6$, OH, F, Cl ou Br ;
$R^3$ et $R^{3'}$, identiques ou différents, peuvent représenter l'hydrogène, F, Cl, Br, méthyle, éthyle, OH, $CF_3$, méthoxy ou phényle, qui peut éventuellement être substitué par un geste choisi dans le groupe F, Cl, Br, méthyle, éthyle, OH, $CF_3$ et méthoxy ;
$R^4$, $R^5$ et $R^6$, identiques ou différents, peuvent représenter l'hydrogène, méthyle ou éthyle,
X peut représenter $NH_2$, NH-(alkyle en $C_1$-$C_6$), N(alkyle en $C_1$-$C_6$)$_2$, où les deux groupes alkyle en $C_1$-$C_6$ peuvent être identiques ou différents, NH-COH, NH-CO(alkyle en $C_1$-$C_6$) ou F ;
A peut représenter $-(CH_2)_3-$, $-CH_2-CH_2-O-$, $-CH_2-O-CH_2-$, $-(CH_2)_4-$, -CH(alkyle en $C_1$-$C_6$)-O-$CH_2$-, $-(CH_2)_2-O-CH_2-$, $-(CH_2)_3-O-$, $-(CH_2)_5-$, $-CH_2-O-(CH_2)_3$, $-(CH_2)_2-O-(CH_2)_2-$, $-(CH_2)_3-O-CH_2-$, $-(CH_2)_4-O-$, $-CH_2-O-CH_2-CH_2-O-$,

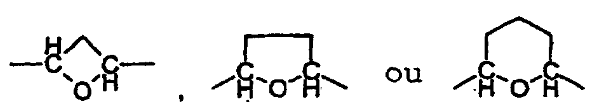

éventuellement sous forme de leurs racémates, de leurs énantiomères, de leurs diastéréoisomères et de leurs mélanges, et éventuellement de leurs sels d'addition d'acide pharmacologiquement acceptables.

**2.** Composés de formule générale 1 selon la revendication 1 où

$R^1$ et $R^2$, identiques ou différents, peuvent représenter l'hydrogène, méthyle, éthyle, méthyloxy, éthyloxy, OH, F, Cl ou Br ;
$R^3$ et $R^{3'}$, identiques ou différents, peuvent représenter l'hydrogène, F, Cl, Br, méthyle, éthyle, OH, $CF_3$, méthoxy ou phényle, qui est substitué par un reste choisi dans le groupe F, Cl, Br et de préférence méthyle ;
$R^4$, $R^5$ et $R^6$, identiques ou différents, peuvent représenter l'hydrogène ou méthyle ;
X peut représenter $NH_2$, NH-(méthyle), N(méthyle)$_2$, NH-(éthyle), N(éthyle)$_2$, NH-COH, NH-COMe ou F ;
A peut représenter $-CH_2-CH_2-O-$, $-CH_2-O-CH_2-$, -CH(méthyle)-O-$CH_2$-, -CH (éthyle)-O-$CH_2$-, -CH(isopropyle) -O-$CH_2$-, - $(CH_2)_2-O-CH_2-$, $-(CH_2)_3-O-$, $-CH_2-O-(CH_2)_3-$, $-(CH_2)_2-O-(CH_2)_2-$, $-(CH_2)_3-O-CH_2-$, - $(CH_2)_4-O-$, $-CH_2-O-CH_2-CH_2-O-$,

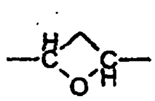 , 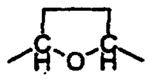 ou 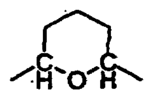

éventuellement sous forme de leurs racémates, de leurs énantiomères, de leurs diastéréoisomères et de leurs mélanges, et éventuellement de leurs sels d'addition d'acide pharmacologiquement acceptables.

**3.** Composés de formule générale 1 selon la revendication 1 ou 2 où

$R^1$ et $R^2$, identiques ou différents, peuvent représenter l'hydrogène ou F ;
$R^3$ et $R^{3'}$, identiques ou différents, peuvent représenter l'hydrogène, F, Cl, Br, $CF_3$ ou méthyle ;
$R^4$, $R^5$ et $R^6$, identiques ou différents, peuvent représenter l'hydrogène ou méthyle ;
X peut représenter $NH_2$, NH-(méthyle), N(méthyle)$_2$, NH-COH, NH-COMe ou F ;
A peut représenter -CH(méthyle)-O-$CH_2$-, $-CH_2-O-CH_2-$ ou

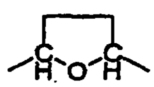

éventuellement sous forme de leurs racémates, de leurs énantiomères, de leurs diastéréoisomères et de leurs mélanges, et éventuellement de leurs sels d'addition d'acide pharmacologiquement acceptables.

**4.** Composés de formule générale 1 selon la revendication 1, 2 ou 3 où

$R^1$ et $R^2$, identiques ou différents, peuvent représenter l'hydrogène ou F ;
$R^3$ et $R^{3'}$, identiques ou différents, peuvent représenter l'hydrogène, F, Cl, Br, $CF_3$ ou méthyle ;
$R^4$, $R^5$ et $R^6$, identiques ou différents, peuvent représenter l'hydrogène ou méthyle ;
X peut représenter $NH_2$, NH-(méthyle) ou NH-COH ;
A peut représenter -CH(méthyle)-O-$CH_2$-, $-CH_2-O-CH_2-$ ou

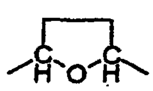

éventuellement sous forme de leurs racémates, de leurs énantiomères, de leurs diastéréoisomères et de leurs mélanges, et éventuellement de leurs sels d'addition d'acide pharmacologiquement acceptables.

5. Composés de formule générale 1 selon la revendication 1, 2 ou 3 où

$R^1$ et $R^2$, identiques ou différents, peuvent représenter l'hydrogène ou F ;
$R^3$ et $R^{3'}$ peuvent représenter l'hydrogène ;
$R^4$, $R^5$ et $R^6$, identiques ou différents, peuvent représenter l'hydrogène ou méthyle ;
X peut représenter F ;
A peut représenter -CH(méthyle)-O-$CH_2$-,

éventuellement sous forme de leurs racémates, de leurs énantiomères, de leurs diastéréoisomères et de leurs mélanges, et éventuellement de leurs sels d'addition d'acide pharmacologiquement acceptables.

6. Préparation pharmaceutique **caractérisée par** une teneur en l'un des composés de formule 1 selon l'une des revendications 1 à 5, outré des adjuvants et supports habituels.

7. Utilisation de l'un des composés de formule 1 selon l'une des revendications 1 à 5 comme médicament.

8. Utilisation de l'un des composés de formule 1 selon l'une des revendications 1 à 5 pour la production d'un médicament pour la prévention ou le traitement de maladies ou troubles où le blocage du canal sodique dépendant de la tension peut présenter une utilité thérapeutique.

9. Utilisation de l'un des composés de formule 1 selon l'une des revendications 1 à 5 pour la production d'un médicament pour la prévention ou le traitement des arythmies, des spasmes, des ischémies cardiaques et cérébrales, des douleurs ainsi que des maladies neurodégénératives.

10. Utilisation de l'un des composés de formule 1 selon l'une des revendications 1 à 5 pour la production d'un médicament pour la prévention ou le traitement de l'épilepsie, de l'hypoglycémie, de l'hypoxie, de l'anoxie, des traumatismes cérébraux, de l'oedème cérébral, de l'attaque cérébrale, de l'asphyxie périnatale, des dégénérescences du cervelet, de la sclérose latérale amyotrope, de la maladie de Huntington, de la maladie d'Alzheimer, de la maladie de Parkinson, de la cyclothymie, de l'hypotonie, de l'infarctus cardiaque, des troubles du rythme cardiaque, de l'angine de poitrine, de la douleur chronique, de la douleur neuropathique ou de l'anesthésie locale.

11. Procédé de production de composés de formule générale 1

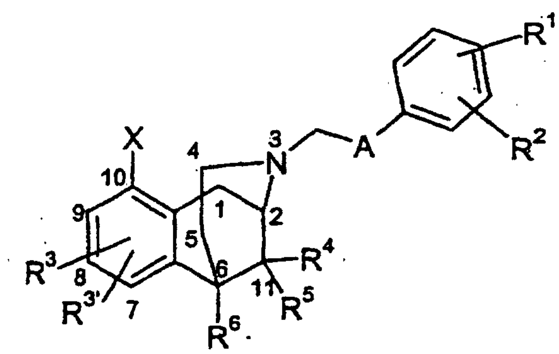

1

où les restes $R^1$, $R^2$, $R^3$, $R^{3'}$, $R^4$, $R^5$, $R^6$ et A peuvent avoir les significations citées dans les revendications 1-5 et X représente $NH_2$, **caractérisé en ce que** des composés de formule générale 3

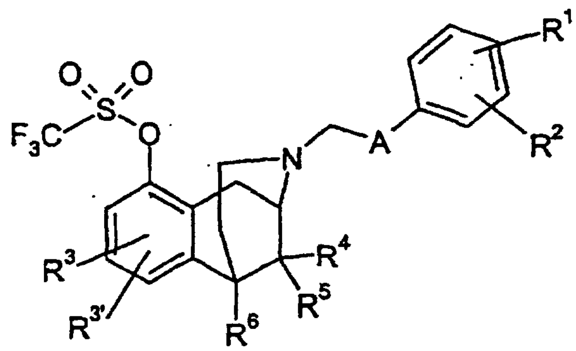

3

où les restes $R^1$, $R^2$, $R^3$, $R^{3'}$, $R^4$, $R^5$, $R^6$ et A peuvent avoir les significations citées dans les revendications 1-5, sont mis à réagir avec une source d'azote dans un solvant aromatique avec catalyse par le palladium.

**12.** Composés intermédiaires de formule générale 3

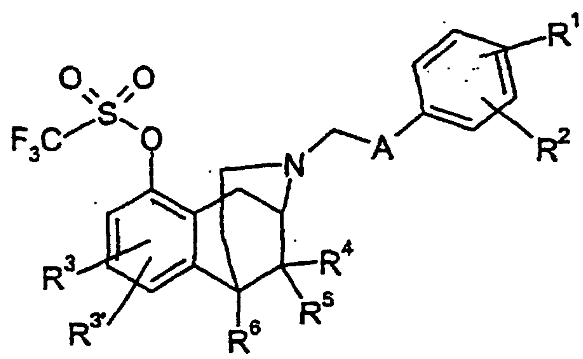

3

où les restes $R^1$, $R^2$, $R^3$, $R^{3'}$, $R^4$, $R^5$, $R^6$ et A peuvent avoir les significations citées dans les revendications 1-5.

**13.** Procédé de production de composés de formule générale 1

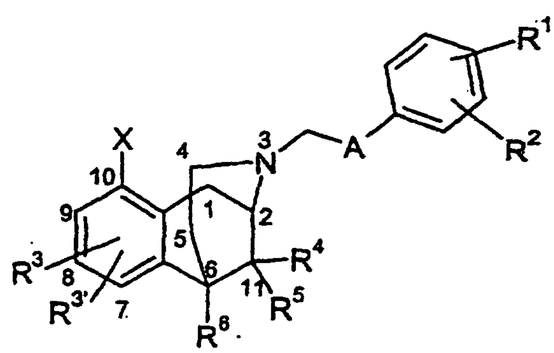

1

où les restes $R^1$, $R^2$, $R^3$, $R^{3'}$, $R^4$, $R^5$, $R^6$ et A peuvent avoir les significations citées dans les revendications 1-5, **caractérisé en ce que** des composés de formule générale 1 dans lesquels X représente $NH_2$

a) sont convertis dans un solvant organique polaire, avec une base et un agent d'alkylation correspondant en

composés de formule générale 1 dans lesquels X représente NH-(alkyle en $C_1$-$C_6$) ou N(alkyle en $C_1$-$C_6$)$_2$, ou bien

b) sont mis à réagir avec des aldéhydes ou des cétones, sous refroidissement, en présence d'acides et les bases de Schiff ou les sels d'iminium ainsi formés de manière intermédiaire sont ensuite réduits avec des hydrures métalliques en composés de formule générale 1 dans lesquels X représente NH-(alkyle en $C_1$-$C_6$) ou N(alkyle en $C_1$-$C_6$)$_2$, ou bien

c) sont mis à réagir en présence d'une base avec un chlorure d'acide ou anhydride d'acide en composés de formule générale 1 dans lesquels X représente NHCO(alkyle en $C_1$-$C_6$), ou bien

d) éventuellement, les composés de formule 1 obtenus selon l'étape c) dans lesquels X représente NHCO (alkyle en $C_1$-$C_6$) sont ensuite réduits avec des hydrures métalliques sous catalyse avec un acide de Lewis en composés de formule générale 1 dans lesquels X représente NH-(alkyle en $C_1$-$C_6$) ou N(alkyle en $C_1$-$C_6$)$_2$, ou bien

e) sont convertis, à température augmentée, par réaction avec l'acide formique, en les composés de formule générale 1 dans lesquels X représente NHCOH, ou bien

f) sont convertis par diazotation de puis chauffage avec $BF_4^-$ en les composés de formule 1 dans lesquels X représente F.